# EUROPEAN PATENT APPLICATION

(11) **EP 3 818 984 A1**
(43) Date of publication of application: **12.05.2021**
(21) Application number: 19831302.5
(22) Date of filing: 08.07.2019
(51) Int. Cl.: A61K 33/44, A61P 25/00, A61P 29/00, A61P 3/00

(54) **GRAPHENE QUANTUM DOT AS THERAPEUTIC AGENT FOR DISEASE ASSOCIATED WITH ABNORMAL FIBRILLATION OR AGGREGATION OF NEUROPROTEIN**

(30) Priority: 06.07.2018 KR 20180079034
(71) Applicant: Biographene Inc., Seoul 06362 (KR); Seoul National University R & DB Foundation, Seoul 08826 (KR)
(72) Inventor: HONG, Byung Hee, Suwon-Si, Gyeonggi-do 16420 (KR); YOO, Je Min, Seoul 06272 (KR)
(74) Representative: Dehns
(86) International application number: PCT/KR2019/008359
(87) International publication number: WO 2020/009551

(57) **Abstract**

The present invention provides a graphene quantum dot as a therapeutic agent for diseases associated with abnormal fibrillation or aggregation of neuroproteins. The graphene quantum dot according to the present invention suppresses α-syn fibrillation or disaggregates already formed α-syn fibrils, and shows the working effect of passing through the blood brain barrier (BBB). Therefore, the graphene quantum dot according to the present invention can be advantageously used as a therapeutic agent for diseases associated with abnormal fibrillation and aggregation of neuroproteins, such as neurodegenerative diseases, inflammatory diseases, and metabolic diseases.

## Description

### TECHNICAL FIELD

The present invention relates to graphene quantum dots and a use thereof, and particularly to graphene quantum dots as a therapeutic agent for neurodegenerative diseases, inflammatory diseases, or metabolic diseases associated with abnormal fibrillization or aggregation of a neuroprotein, and a use thereof.

### BACKGROUND ART

α-synuclein (a-syn) is a protein that is abundant in the human brain, mainly found at the ends of nerve cells (neurons), and known to interact with phospholipids and proteins within these structures. The abnormal aggregation of α-syn leads to the formation of α-syn fibrils (a-syn PFFs), and it is known that synucleinopathy, i.e., a neurodegenerative disease, metabolic diseases, and the like are caused by these α-syn fibrils. Therefore, the development of a therapeutic agent capable of treating neurodegenerative diseases, metabolic diseases, and the like by inhibiting the fibrillization of α-syn or disaggregating already formed α-syn fibrils is actively ongoing (Korea Patent Publication No. 10- 2018-0081465). However, there is still no practically usable therapeutic agent showing a remarkable effect on synucleinopathy, and therefore there is an urgent need to develop a therapeutic agent therefor.

Meanwhile, graphene quantum dots have so far been used only for drug delivery systems. However, the inventors of the present invention found that specific graphene quantum dots have an acting effect of inhibiting fibrillization or disaggregating already formed α-syn fibrils, and thus completed the present invention.

### DESCRIPTION OF EMBODIMENTS

### TECHNICAL PROBLEM

An object of the present invention is to provide graphene quantum dots that exhibit therapeutic activity in diseases associated with the abnormal fibrillization or aggregation of a neuroprotein.

Another object of the present invention is to provide a use of graphene quantum dots according to the present invention.

However, technical problems to be solved by the present invention are not limited to the above-described technical problems, and other unmentioned technical problems will become apparent from the following description to those of ordinary skill in the art to which the present invention pertains.

### TECHNICAL SOLUTION

The present invention provides graphene quantum dots having negatively charged surfaces, an average diameter of 0.5 nm to 10 nm, an average height of 0.1 nm to 3 nm, and a ratio (wt%) of carbon to oxygen of 4.0-6.5: 3.0-6.0. The average diameter may range, more preferably, from 1 nm to 5 nm, and the average height may range from 0.5 nm to 2.5 nm, but the present invention is not limited thereto.

In one embodiment of the present invention, the graphene quantum dots may include, preferably, carboxyl groups as terminal functional groups. Preferably, the absorbance ratio of a -C=O peak of the carboxyl groups to an aromatic -C=C- peak in an FT-IR spectrum is 1:1 or more, and more preferably, the absorbance ratio may range from 1:1 to 2:1.

In another embodiment of the present invention, the -C=O peak may appear at 1,700 cm⁻¹ to 1,750 cm⁻¹, and the aromatic -C=C- peak may appear at 1,600 cm⁻¹ to 1,650 cm⁻¹.

In another embodiment of the present invention, the graphene quantum dots may inhibit α-syn fibrillization or disaggregate already formed α-syn fibrils, and penetrate the blood-brain barrier.

In another embodiment of the present invention, the graphene quantum dots have no cytotoxicity, and can be stably excreted from the body through urine.

The present invention also provides a pharmaceutical composition for the prevention or treatment of diseases associated with the abnormal fibrillization or aggregation of a neuroprotein, including the graphene quantum dots as an active ingredient.

In one embodiment of the present invention, the diseases associated with abnormal fibrillization or aggregation of a neuroprotein are neurodegenerative diseases, inflammatory diseases, or metabolic diseases. The neurodegenerative diseases may be Alzheimer's disease, Parkinson's disease, Huntington's disease, Lou Gehrig's disease, dementia, stroke, amyloidosis, fibrosis, encephalopathy, multiple sclerosis, and the like, the inflammatory diseases may be erythema, atopy, rheumatoid arthritis, Hashimoto's thyroiditis, pernicious anemia, Edison's disease, type 1 diabetes, lupus, chronic fatigue syndrome, fibromyalgia, hypothyroidism, hyperthyroidism, scleroderma, Behcet's disease, inflammatory bowel disease, myasthenia gravis, Meniere's syndrome, Guilian-Barre syndrome, Sjogren's syndrome, endometriosis, psoriasis, leukoplakia, systemic scleroderma, ulcerative colitis, and the like, and the metabolic diseases may be diabetes, hypertension, hyperlipidemia, dyslipidemia, non-alcoholic fatty liver, and the like, but the diseases are not limited thereto as long as they are diseases caused by the abnormal fibrillization or aggregation of α-syn.

The present invention also provides a method of preventing or treating diseases associated with the abnormal fibrillization or aggregation of a neuroprotein, the method including administering, to an individual, a composition including the graphene quantum dots as an active ingredient.

The present invention also provides a use of the composition including the graphene quantum dots as an active ingredient for the prevention or treatment of diseases associated with the abnormal fibrillization or aggregation of a neuroprotein.

### ADVANTAGEOUS EFFECTS OF INVENTION

Graphene quantum dots according to the present invention can inhibit the formation of α-syn fibrils or disaggregate already formed α-syn fibrils, and can exhibit not only an acting effect of passing through the blood-brain barrier but also no cytotoxicity. Thus, the graphene quantum dots according to the present invention can be effectively used as a therapeutic agent for neurodegenerative diseases, inflammatory diseases, metabolic diseases, and the like associated with the abnormal fibrillization or aggregation of a neuroprotein.

### BRIEF DESCRIPTION OF DRAWINGS

FIGS. 1A to 1K illustrate analysis results showing the effect of graphene quantum dots on inhibiting α-syn fibrillization and disaggregating already formed α-syn fibrils.
FIGS. 2A to 2H illustrate detailed analysis results showing the interactions between graphene quantum dots and already formed α-syn fibrils during a fibril disaggregation process.
FIGS. 3A to 3J illustrate *in-vitro* results showing the effect of graphene quantum dots on neuronal death by α-syn PFFs and intercellular propagation of α-syn fibrils between neurons.
FIGS. 4A to 4O illustrate results showing the effect of graphene quantum dots on pathological phenomena induced by α-syn PFFs *in vivo.*
FIG. 5 is a schematic view illustrating an overview of the therapeutic effect of graphene quantum dots on the pathogenesis of Parkinson's disease.
FIGS. 6A to 6G illustrate results of analyzing the synthesis of graphene quantum dots, biotinylation, and binding between biotin-graphene quantum dots and α-syn fibrils.
FIG. 7 illustrates results showing the effect of graphene quantum dots on the disaggregation of α-syn fibrils.
FIG. 8 illustrates results showing the effect of graphene quantum dots on the disaggregation of α-syn PFFs.
FIG. 9 illustrates ¹H-¹⁵N HSQC spectrum analysis results of synthesized graphene quantum dots.
FIGS. 10A to 10G illustrate results showing the effect of graphene quantum dots on neuronal death and limited neurite growth induced by α-syn PFFs.
FIGS. 11A to 11J illustrate results showing the effect of graphene quantum dots on mitochondrial dysfunction and active oxygen generation induced by α-syn PFFs.
FIGS. 12A to 12H illustrate results showing the effect of graphene quantum dots on the induction of primary neurocytotoxicity and pathological phenomena at various treatment time points, induced by α-syn PFFs and results related to live cell imaging.
FIGS. 13A to 13N illustrate results showing the blood-brain barrier permeability of graphene quantum dots.
FIG. 14 illustrates results showing the effect of graphene quantum dots on glial cell activation induced by α-syn PFFs in the substantia nigra in the midbrain.
FIGS. 15A to 15C illustrate results showing the effect of graphene quantum dots on glial cell activation in the brain stem of hA53T α-syn transgenic mice.
FIGS. 16A to 16E illustrate results of *in vitro* and *in vivo* toxic effects of graphene quantum dots over a long period of time.
FIGS. 17A to 17H illustrate results of comparing nano-GOs and rGQDs for α-syn PFFs.

### BEST MODE

The inventors of the present invention have found that graphene quantum dots are capable of exhibiting therapeutic activity in diseases associated with the abnormal fibrillization or aggregation of a neuroprotein, and thus completed the present invention. The inventors of the present invention confirmed that the graphene quantum dots have an average diameter of 0.5 nm to 10 nm, an average height of 0.1 nm to 3 nm, and a ratio (wt%) of carbon to oxygen of 4.0-6.5: 3.0-6.0, and the surfaces thereof are negatively charged. It was also confirmed that the graphene quantum dots not only exhibited no toxicity to cells and tissues, but could also effectively prevent and treat the abnormal fibrillization or aggregation of a neuroprotein, that is, α-syn. It was also confirmed that, since the graphene quantum dots can penetrate the blood-brain barrier, the graphene quantum dots exhibit remarkable therapeutic effects on various neurodegenerative diseases, inflammatory diseases, metabolic diseases, and the like caused by the abnormal fibrillization or aggregation of α-syn.

Throughout the present specification, the term "graphene" means that a plurality of carbon atoms are covalently linked to each other to form a polycyclic aromatic molecule, and the carbon atoms linked by the covalent bonds form a 6-membered ring as a basic repeating unit, but may also further include a 5-membered ring and/or a 7-membered ring.

Throughout the present specification, the term "graphene quantum dots (GQDs)" refers to graphene having nano-sized fragments.

Throughout the present specification, the term "reduced graphene quantum dots" refers to oxidized graphene having a reduced oxygen atom ratio through a reduction process, and may be abbreviated as "rGQDs".

Throughout the present specification, the term "oxidized graphene" is also called graphene oxides, and may be abbreviated as "GOs". The oxidized graphene may include a structure in which an oxygen atom-containing functional group such as a carboxyl group, a hydroxyl group, an epoxy group, or the like is bound onto graphene, but the present invention is not limited thereto.

Throughout the present specification, the term "nano-graphene oxides" may be abbreviated as "nano-GOs" as nanoscale graphene oxides having an average diameter of 15 nm or more and an average height of 5 nm or more.

Hereinafter, graphene quantum dots according to the present invention and a use thereof will be described in detail.

### Graphene Quantum Dots

The present invention provides graphene quantum dots capable of exhibiting therapeutic activity in diseases associated with the abnormal fibrillization or aggregation of a neuroprotein.

Specifically, the graphene quantum dots according to the present invention have negatively charged surfaces and have an average diameter of about 0.5 nm to about 10 nm and an average height of about 0.1 nm to about 3 nm. In addition, the graphene quantum dots have a structure in which the ratio (wt%) of carbon to oxygen ranges from 4.0-6.5: 3.0-6.0.

Since the surfaces of the graphene quantum dots according to the present invention are negatively charged, they can bind to the ends of α-syn fibrils, thereby inhibiting the formation of α-syn fibrils or disaggregating already formed α-syn fibrils into monomers. Through such a mechanism, the graphene quantum dots according to the present invention can exhibit therapeutic activity in diseases associated with the abnormal fibrillization or aggregation of a neuroprotein, for example, Parkinson's disease and dementia with Lewy bodies.

According to the present invention, the graphene quantum dots of the present invention may include oxygen atom-containing functional groups such as carboxyl groups, ketone groups, aldehyde groups, hydroxyl groups, and epoxy groups as terminal functional groups. In particular, the graphene quantum dot may contain a carboxyl group exhibiting a negative charge as a major functional group.

According to one embodiment of the present invention, in a FT-IR spectrum of the graphene quantum dots of the present invention, the absorbance ratio of a -C=O peak of the carboxyl groups and an aromatic -C=C- peak is about 1:1 or more. In particular, the absorbance ratio may be about 1:1 to about 30:1, about 1:1 to about 20:1, about 1:1 to about 1:15, about 1:1 to about 1:10, about 1:1 to about 1:7, about 1:1 to about 1:5, about 1:1 to about 1:3, or about 1:1 to about 2:1. However, the absorbance ratio is characterized by having a lower limit of about 1:1 and is not limited as long as it is about 1:1 or more.

In the present invention, the -C=O peak refers to a peak appearing at about 1,700 cm⁻¹ to about 1,750 cm⁻¹, and the aromatic -C=C- peak refers to a peak appearing at about 1,600 cm⁻¹ to about 1,650 cm⁻¹.

The graphene quantum dots according to the present invention have an average diameter (lateral size) of about 0.5 nm to about 10 nm and an average height of about 0.1 nm to about 3 nm, and exhibit a small nanoscale size.

Specifically, the average diameter of the graphene quantum dots according to the present invention may range from about 0.5 nm to about 10 nm, about 0.7 nm to about 7 nm, about 0.8 nm to about 6 nm, or about 1 nm to about 5 nm.

In addition, the average height of the graphene quantum dots according to the present invention may range from about 0.1 nm to about 3 nm, about 0.2 nm to about 3 nm, about 0.3 nm to about 3 nm, about 0.5 nm to about 3 nm, or about 0.5 nm to about 2.5 nm.

The graphene quantum dots of the present invention as described above can inhibit the fibrillization of an α-syn monomer or disaggregate already formed α-syn fibrils using negative charges formed on the surfaces thereof, and have no toxicity in the body. In addition, the graphene quantum dots of the present invention can easily penetrate the blood-brain barrier (BBB) due to the small nanoscale size thereof. In addition, the graphene quantum dots of the present invention was not found to have the problem of being accumulated in the body.

Therefore, the graphene quantum dots according to the present invention may be effectively used as a therapeutic agent for diseases associated with the abnormal fibrillization or aggregation of a neuroprotein.

### Use of Graphene Quantum Dots

The present invention provides a use of the graphene quantum dots according to the present invention.

As described above, the graphene quantum dots of the present invention can penetrate the blood-brain barrier to inhibit α-syn fibrillization or disaggregate already formed α-syn fibrils into monomers. Therefore, the graphene quantum dots according to the present invention may be effectively used as a therapeutic agent for diseases associated with the abnormal fibrillization or aggregation of a neuroprotein.

An embodiment of the present invention provides a pharmaceutical composition for the prevention or treatment of diseases associated with the abnormal fibrillization or aggregation of a neuroprotein, including graphene quantum dots according to the present invention as an active ingredient.

In the present invention, the diseases associated with the abnormal fibrillization or aggregation of a neuroprotein are neurodegenerative diseases, inflammatory diseases, or metabolic diseases.

Specifically, the neurodegenerative diseases may be selected from the group consisting of Alzheimer's disease, Parkinson's disease, Huntington's disease, Lou Gehrig's disease, dementia, stroke, amyloidosis, fibrosis, encephalopathy, and multiple sclerosis. However, the present invention is not limited thereto.

In addition, the inflammatory diseases may be selected from the group consisting of erythema, atopy, rheumatoid arthritis, Hashimoto's thyroiditis, pernicious anemia, Edison's disease, type 1 diabetes, lupus, chronic fatigue syndrome, fibromyalgia, hypothyroidism, hyperthyroidism, scleroderma, Behcet's disease, inflammatory bowel disease, myasthenia gravis, Meniere's syndrome, Guilian-Barre syndrome, Sjogren's syndrome, endometriosis, psoriasis, leukoplakia, systemic scleroderma, and ulcerative colitis. However, the present invention is not limited thereto.

In addition, the metabolic diseases may be selected from the group consisting of diabetes, hypertension, hyperlipidemia, dyslipidemia, and non-alcoholic fatty liver. However, the present invention is not limited thereto.

The pharmaceutical composition of the present invention may further include one or more pharmaceutically acceptable carriers, in addition to the graphene quantum dots according to the present invention for administration. Pharmaceutically acceptable carriers can be saline, sterile water, Ringer's solution, buffered saline, a dextrose solution, a maltodextrin solution, glycerol, ethanol, and a mixture of two or more of these components. As necessary, other general additives such as antioxidants, buffers, and bacteriostatic agents may be added. In addition, diluents, dispersants, surfactants, binders, and lubricants may be additionally added for formulation into pills, capsules, granules, tablets, or injectable formulations such as aqueous solutions, suspensions, and emulsions. Thus, the pharmaceutical composition of the present invention may be patches, liquids, pills, capsules, granules, tablets, suppositories, or the like. These preparations can be prepared by a conventional method used for formulation in the art or by a method disclosed in Remington's Pharmaceutical Science (latest edition), Mack Publishing Company, Easton PA, and various preparations may be formulated according to each disease or ingredient.

The pharmaceutical composition of the present invention may be administered orally or parenterally (e.g., intravenous administration, subcutaneous administration, intraperitoneal administration, or topical administration) according to a target method, and a suitable dose thereof varies depending on the body weight, age, gender, and health condition of a patient, diet, administration time, administration method, excretion rate, the severity of a disease, and the like. A daily dosage of the graphene quantum dots according to the present invention ranges from about 1 mg/kg to about 1,000 mg/kg, preferably 5 mg/kg to 100 mg/kg, and may be administered once to several times a day.

The pharmaceutical composition of the present invention may further include one or more active ingredients exhibiting the same or similar efficacy, in addition to the graphene quantum dots according to the present invention.

Another embodiment of the present invention provides a method of preventing or treating diseases associated with the abnormal fibrillization or aggregation of a neuroprotein, the method including administrating a therapeutically effective amount of the graphene quantum dots according to the present invention.

The term "therapeutically effective amount" used herein refers to the amount of the graphene quantum dots that are effective in the prevention or treatment of diseases associated with the abnormal fibrillization or aggregation of a neuroprotein.

The method of preventing or treating diseases associated with the abnormal fibrillization or aggregation of a neuroprotein according to the present invention includes not only dealing with the disease itself before the onset of symptoms, but also inhibiting or avoiding the symptoms thereof by administering the graphene quantum dots. In the management of a disease, the prophylactic or therapeutic dose of a specific active ingredient will vary depending on the nature and severity of the disease or condition, and administration route of the active ingredient. The dose and frequency of dose will vary depending on the age, body weight, and response of an individual patient. A suitable dosage regimen may be readily selected by those of ordinary skill in the art in consideration of these factors. In addition, the method of preventing or treating diseases associated with the abnormal fibrillization or aggregation of a neuroprotein according to the present invention may further include administering a therapeutically effective amount of an additional active agent useful for the treatment of diseases, along with the graphene quantum dots, and the additional active agent may exhibit a synergistic effect or an auxiliary effect together with the graphene quantum dots.

Another embodiment of the present invention provides a use of the graphene quantum dots according to the present invention for preparing a drug for the treatment of diseases associated with the abnormal fibrillization or aggregation of a neuroprotein. The graphene quantum dots for preparing a drug may be mixed with acceptable adjuvants, diluents, carriers, and the like, and may be prepared as a composite preparation together with other active agents, thereby having a synergistic effect of active ingredients.

The descriptions provided in the use, composition, and treatment method of the present invention equally apply unless otherwise contradicted.

The term "prevention" as used herein means all actions that inhibit diseases associated with the abnormal fibrillization or aggregation of a neuroprotein or delay the onset thereof via administration of the composition according to the present invention.

The term "treatment" as used herein means all actions that alleviate or beneficially change symptoms due to diseases associated with the abnormal fibrillization or aggregation of a neuroprotein via administration of the composition according to the present invention.

In the present specification, "individual" refers to a subject to which the composition of the present invention can be administered, and there is no limitation on the subject.

Hereinafter, exemplary examples will be described to aid in understanding of the present invention. However, these examples are provided only to facilitate the understanding of the present invention and are not intended to limit the scope of the present invention.

### Example: Preparation of Graphene Quantum Dots (GQDs)

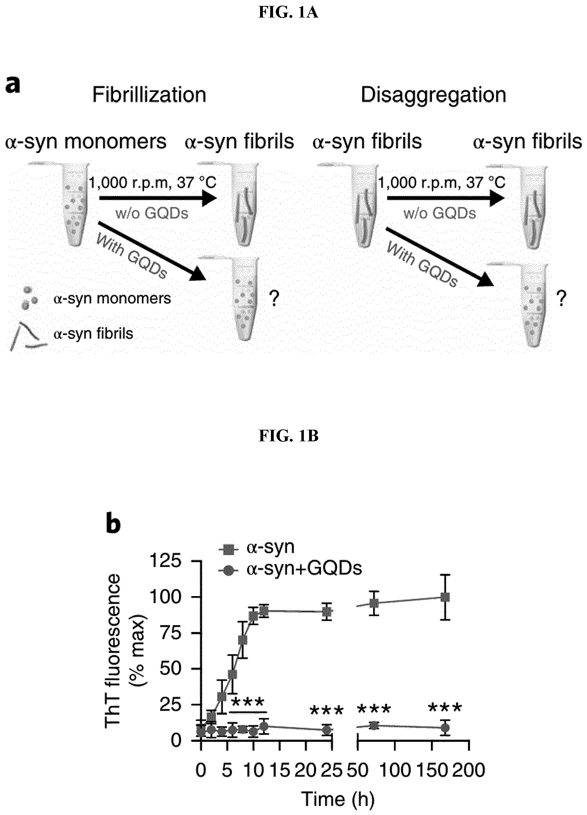

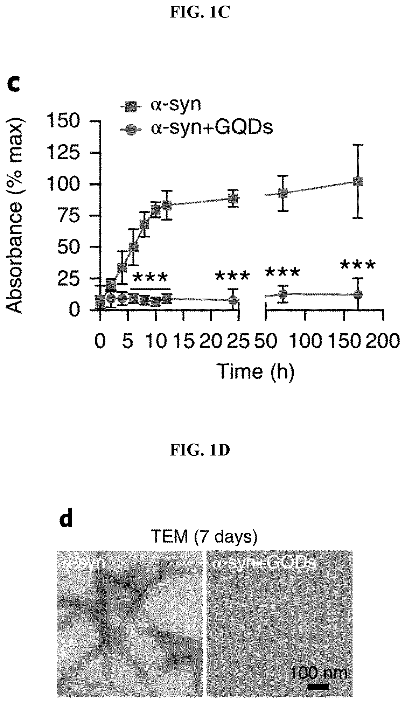

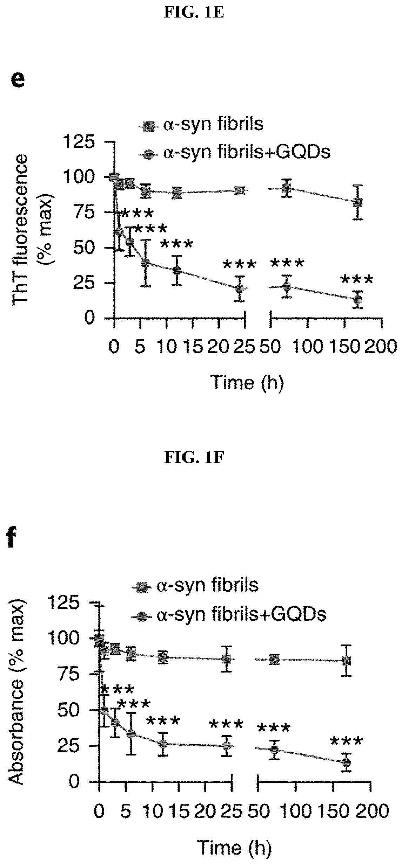

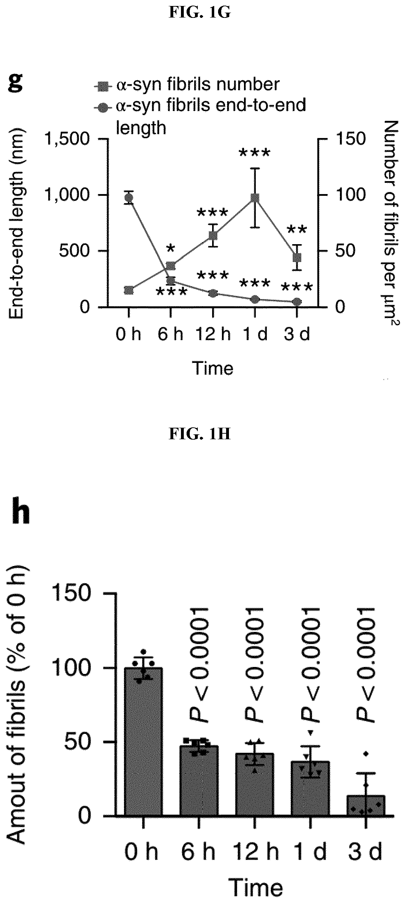

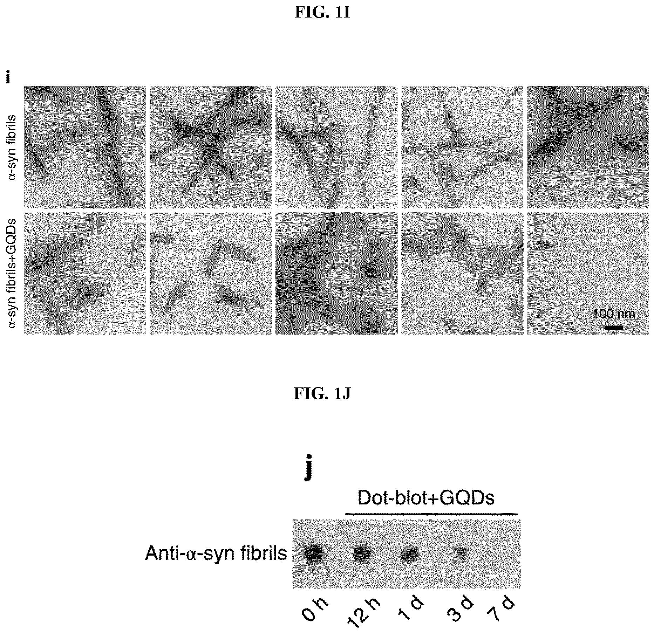

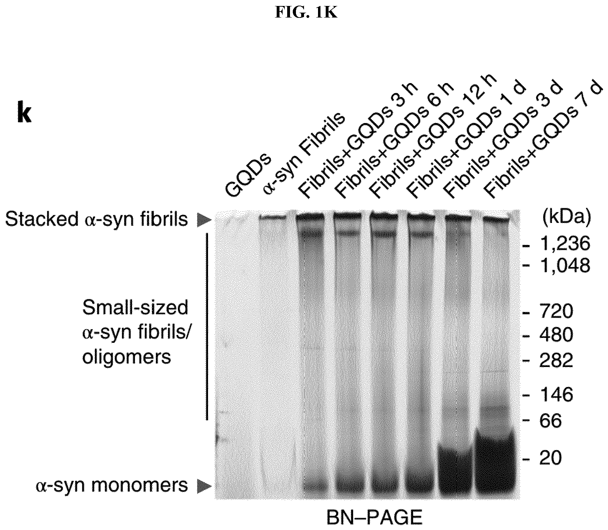

Carbon fiber (0.9 g) was added to a mixed solution of sulfuric acid (300 ml) and nitric acid (100 ml), followed by heating at 80 °C for 24 hours (thermo-oxidation process, see FIG. 1). Then, the resulting solution was diluted with deionized water, followed by dialysis with a regenerated nitrocellulose membrane (Cat#: 06-680-2G; MWCO 1,000 Dalton; Fisher Scientific) to completely remove the acid and excess carbon fragments. Then, the GQD solution was vacuum-filtered with a porous inorganic membrane filter (Cat#: 6809-5002; Whatman-Anodisc 47; GE Healthcare) to remove large particles. Thereafter, the solution was rotary evaporated to obtain powder-type graphene quantum dots (GQDs). The obtained graphene quantum dots contained both the graphitic domain and edge functional groups, and thus were subjected to elemental analysis (EA) of main elemental components including nitrogen (N), carbon (C), hydrogen (H), sulfur (S), and oxygen (O). The elemental analysis was performed using an Elemental analyzer Flash2000 (Thermo Fisher Scientific). The results thereof are shown in Table 1.

**[Table 1]**

| | | | | | | | |
|---|---|---|---|---|---|---|---|
| Graphene Quantum dots | Element | N | C | H | S | O | Total |
| | mol | 0.10 | 2.80 | 1.29 | 0.07 | 1.62 | - |
| | wt% | 2.12 | 52.21 | 2.00 | 3.31 | 40.36 | 100 |

As shown in Table 1, it was confirmed that the graphene quantum dots of the present invention had a ratio (wt%) of carbon to oxygen, which are core elements, of 5.2±1: 4.0±1.

### Comparative Example: Preparation of nano-GOs and rGQDs

To prepare nano-GOs, pristine graphene oxides (GOs) were synthesized by an improved Hummer method according to the conventional literature. The obtained GO powder was added to deionized water at a concentration of 10 mg/ml, followed by tip-ultrasonic treatment for 3 hours to obtain nano-GOs.

The reduction of GQDs was carried out by an autoclave-based hydrothermal method at 200 °C for 2 hours to prepare rGQDs.

The products were analyzed by FT-IR spectroscopy and AFM analysis, and there was practically no difference between rGQDs and pristine GQDs in AFM results.

### Experimental Method

### 1. AFM Imaging

For AFM imaging, each sample (10 µg/ml) and α-syn fibrils (10 µg/ml) were added dropwise onto a 1 cm² silicon oxide substrate and dried at room temperature. Analyses were performed with an XE-100 AFM (Park Systems) under a non-contact mode (scan size: 25 µm² and scan rate: 0.8 Hz). Images were acquired with the XE data collection program (XEP 1.8.0).

### 2. FT-IR Measurement

Samples were completely dried in a high-vacuum desiccator and prepared by a conventional KBr pellet method. Then, measurement was performed using a Nicolet 6700 FT-IR spectrometer (Thermo Scientific) under conditions of a scan number of 32 and a wavenumber range of 4,000 cm⁻¹ to 40 cm⁻¹.

### 3. Thioflavin T (ThT) Fluorescence and Turbidity Analysis

For ThT assay, 50 µl of each sample was centrifuged at 16,000 x g for 30 minutes. Then, the pellet was re-suspended in 200 µl of 25 µM ThT (Cat#: T3516; Sigma Aldrich) dissolved in a 10 mM glycine buffer (pH 9.0). ThT fluorescence was measured at 482 nm (excitation at 440 nm) using a fluorescence spectrophotometer.

For turbidity assay, α-syn fibrils were diluted at a ratio of 1/10 using phosphate buffered saline (PBS). Then, the diluted α-syn fibrils were transferred to a Corning 96-well plate, and the absorbance intensity at 360 nm was measured using a microplate multi-reader to evaluate the turbidity of each sample.

### 4. TEM Imaging

Each sample was adsorbed onto a glow-discharged 400 mesh carbon-coated copper grid (manufactured by EMS) for 2 minutes. Then, three drops of a 50 mM Tris-HCl (pH 7.4) solution were quickly added to the grid for washing, and then two drops of 0.75% uranyl formate were continuously flowed for 30 seconds each. The stain was removed using #1 Whatman filter paper. Each sample was sufficiently dried before measurement, and digitized images were acquired using an electron microscope Phillips CM 120 TEM operating at 80 kV and AMT's ER-80 CCD (8 megapixels). In the case of neurons, primary cultured cortical-derived neurons were cultured at a density of 100,000 cells/well on a 35 mm dish coated with poly-D-lysine. The neurons were treated with 1 µg/ml of PFFs with or without 1 µg/ml of graphene quantum dots on DIV 10. After 7 days, the neurons were washed with PBS containing 1% sodium nitrite (pH 7.4), and then fixed using a fixing solution consisting of 3%(v/v) paraformaldehyde, 1.5%(v/v) glutaraldehyde, 100 mM cacodylate, and 2.5% (v/v) sucrose (pH 7.4), and post-fixed for 1 hour. Images were acquired with a Phillips EM 410 TEM equipped with a Soft Imaging System Megaview III digital camera.

### 5. Dot-blot Analysis

Each sample was loaded onto a pre-soaked nitrocellulose membrane (pore size: 0.45 µm) using a Bio-Dot microfiltration device (Cat#: 170645; Bio-Rad), and adhered to the membrane using negative pressure. Each membrane was washed with Tris-buffered saline, and then blocked with Tween-20-containing Tris-buffered saline containing 5% skim milk powder. Each sample was allowed to bind to a conformation-specific anti-α-syn filament antibody (Cat#: ab209538; 1:1, Abcam) overnight at 4 °C, and then cultured at room temperature for 1 hour along with an HRP-conjugated secondary antibody obtained from rabbit (GE Healthcare). Then, each membrane was washed several times with Tris-buffered saline, imaged using an ECL solution, and analyzed using ImageJ software.

### 6. BN-PAGE and SDS-PAGE

For BN-PAGE, α-syn fibrils and α-syn PFFs were prepared using a NativePAGETM sample preparation kit (Cat#: BN2008; Life Technologies), and subjected to electrophoresis using NativePAGETM Novex 4-16% bis-Tris gel (Cat#: BN1002Box; Life Technologies) at 200 V for 90 minutes. A cathode buffer solution contains 50 mM tricine, 15 mM Bis-Tris, and 0.02% Brilliant Blue G (pH 7.0), and an anode buffer solution consists of 50 mM Bis-Tris (pH 7.0). Gels were stained using SilverQuestTM Silver Stain Kit (Cat#: LC6070; Life Technologies) in accordance with the manufacturer's instructions.

For SDS-PAGE, 10 DIV cortical-derived neurons were treated with α-syn PFFs (5 µg/ml) in the presence or absence of 5 µg/ml graphene quantum dots for 7 days. Available proteins of neurons were prepared using a cocktail of 1% TX-100, a protease and phosphatase inhibitor in PBS solution (Cat#: PPC1010; Sigma Aldrich) at 4 °C. Then, the lysate was sonicated at 4 °C at 12,000 x g for 30 minutes. The pellet was washed several times and suspended in PBS containing 2% SDS for insoluble protein production. The lysate was diluted using a 2X Laemmli sample buffer (Cat#: 1610737, Bio-Rad). Subsequently, 20 µg of proteins were loaded onto NovexTM 8-16% Tris-glycine gel (Cat#: XP08160BOX, Life Technologies), and subjected to electrophoresis at 130 V for 85 minutes. Then, the proteins were transferred to a nitrocellulose membrane, blocked with physiological saline with Tris containing 0.1% Tween-20, containing 5% skim milk powder for 1 hour, conjugated with an anti-pS129-α-syn (Cat#: ab59264, 1:1,000, Abcam), SNAP25 (Cat#: 111-002, 1:2,000, Synaptic Systems) or VAMP2 (Cat#: ab3347, 1:1,000, Abcam) antibody overnight at 4 °C, and then cultured with an HRP-conjugated secondary antibody (GE Healthcare) isolated from rabbits or mice at room temperature for 1 hour. The blots were visualized with an ECL solution and analyzed using ImageJ software.

### 7. Preparation of Sonicated α-syn PFFs

α-syn PFFs were prepared according to a conventional method reported by Volpicelli-Daley et al. (Nat Protoc 9, 2135-2146, doi: 10.1038/nprot.2014.143 (2014)). Full-length mouse recombinant α-syn was cloned into the ampicillin-resistant bacterial expression vector pRK172. Then, a plasmid was transformed into BL21(DE3)RIL-competent *E*. *coli* (Cat#: 23045, Life Technologies) and used. After bacterial culture, the α-syn monomers were isolated according to the literature mentioned above and purified purely through several purification processes including anion exchange, dialysis, and size exclusion chromatography. *In vitro* α-syn fibrils were aggregated in an Eppendorf orbital mixer (Cat#: 538400020) while being stirred at 37 °C and 1,000 rpm for 7 days. Fragments of the α-syn fibrils were made by treatment with a total of 60 pulses (~0.5 sec each) at 20% intensity using an ultrasonic device with a 1/8" probe-sonicator. The fragments were cultured with primary cultured neurons at 37 °C for 7 days, and then naturally converted into mature fibrils in neurons, and it was confirmed that they exhibited toxicity to cells.

### 8. Biotinylation of Graphene Quantum Dots and Binding Assay

To introduce carboxyl groups onto graphene quantum dots for functionalization, 50 mg of graphene quantum dots were dissolved in a conjugation buffer (pH 4.7). Subsequently, 12.5 mg of EDC reagent (N-3 (dimethylaminopropyl)-N-ethylcarbodiimide hydrochloride, Cat#: 03449, Sigma Aldrich) was added to introduce carboxyl groups to the graphene quantum dots. After stirring for 1 hour, 25 mg of EZ-Link amine-PEG3-biotin (Cat#: 21347, Thermo Scientific) was added to EDC-activated graphene quantum dots to cause the formation of amide bonds between the graphene quantum dots and biotin for 12 hours. The solution was dialyzed against a regenerated nitrocellulose membrane (Cat#: 06-680-2G, MWCO 1,000 Dalton, Fisher Scientific) to remove unreacted biotin and the EDC reagent. Then, the solution was used in a rotary concentrator to obtain a final product in a powder form. For the assay of binding between graphene quantum dots and α-syn fibrils, 5 mg/ml of α-syn fibrils were incubated with 5 mg/ml of biotinylated graphene quantum dots and streptavidin-conjugated 0.8 nm gold nanoparticles (Cat#: 800.099, Aurion) for 1 hour. Thereafter, the streptavidin-conjugated ultra-small gold particles bound to biotinylated-graphene quantum dots with high affinity were enhanced with a GoldEnhanceTM EM Plus solution (Cat#: 2114, Nanoprobes) for 5 minutes. The unreacted solution was removed by spin column with 100 kDa MWCO (Cat#: UFC510024, Millipore-Sigma), followed by TEM analysis.

### 9. Purification of ¹⁵N-labeled α-syn

For α-syn overexpression, the α-syn gene cloned into the pRK172 vector was transformed into *E. coli* BL21 (DE3). For the preparation of isotope-labeled α-syn, cells were grown with 100 µg of ampicillin in an M9 medium containing 0.5 g/l L of ¹⁵NH₄Cl per liter and 1 g of ¹³C glucose (Cambridge Isotope Laboratory Inc., Andover, MA) per liter at 37 °C. After induction with isopropyl β-D-thiogalactopyranoside (IPTG), the heat-treated cell lysate was continuously purified using DEAE-Sephacel anion exchange, Sephacryl S-200 size exclusion, and S-Sepharose cation chromatography. The purified α-syn was dialyzed 3 times with 12 L of a fresh 20 mM 2-(N-morpholino)ethanesulfonic acid (MES) buffer (pH 6.5), and dispensed in aliquots of 1 mg/ml concentration and stored at -80 °C. Immediately before the experiment, each sample was concentrated to 5 mg/ml using a Nanosep 10 K membrane (Pall Gelman, Germany) at 4 °C.

### 10. NMR Spectroscopic Analysis

Detailed NMR studies on the interaction between α-syn and graphene quantum dots were conducted using a 950 MHz spectrometer (Bruker, Germany) equipped with a cryo-genic probe. Uniformly classified ¹⁵N-labeled α-syn (5 mg/ml, 100 µl) was reacted with GQDs (5 mg/ml, 100 µl) at 37 °C for 3 days while being stirred at 1,000 rpm. For ¹H-¹⁵N HSQC measurement, a ¹⁵N-labeled α-syn sample was prepared using a 10% 20 mM MES buffer (pH 6.5). The ¹H-¹⁵N HSQC spectra of α-syn and α-syn reacted with graphene quantum dots were obtained at 37 °C. The obtained data was processed with NMRPipe24 and analyzed with Sparky25.

### 11. Simulation Details

In order to investigate the interaction between graphene quantum dots and α-syn fibrils at the molecular level, a 200-ns molecular dynamics (MD) simulation was performed with Gromacs 5.1. The initial structure of the hydrophobic NAC domain (residues 71 to 82) of α-syn was applied from the ssNMR structure (PDB ID: 2N0A) with the CHARMM force field, and the structure of the graphene quantum dots was designed with CGenFF in accordance with the protocols of https://cgenff.paramchem.org.

### 12. CD Measurement

α-syn fibrils (5 mg/ml, 100 µl) were mixed with an aqueous graphene quantum dot solution (5 mg/ml, 100 µl), and depolymerized under shaking culture at 37 °C and 1,000 rpm for 7 days. For far-UV CD measurement, each sample was diluted (1/2) with distilled water. CD spectra between 190 nm and 260 nm were measured at 0.5 nm intervals using a J-815 spectrophotometer (Jasco, Japan) and a 0.2 mm-long quartz cuvette. The spectrum of the buffer solution was subtracted from the sample spectra. CD signals were normalized to the mean residue ellipticity [θ], with units of deg cm²/dmol. The fractional secondary structure contents of α-syn fibrils and depolymerized α-syn fibrils were calculated using the algorithm of CONTIN/LL provided on the DichroWeb online server. For calculations using CONTIN/LL, DichroWeb's reference set 7 was used and optimized for the wavelength range between 190 nm and 240 nm.

### 13. Primary Neuron Culture

Primary neurons derived from a mouse cerebral cortex were prepared using 15-day-old C57BL/6 mouse embryos. The isolated neurons were maintained in a culture medium consisting of Neurobasal Media (Cat#: 21103049, Life Technologies) containing a B27 supplement (Cat#: 17504044, Life Technologies) and L-glutamine (Cat#: 25030149, Life Technologies), and plated on a culture dish coated with 50 µg/ml of poly-D-lysine (Cat#: P6407, Sigma Aldrich). The culture medium was replaced twice a week and the plate was maintained in a 7 % CO₂ incubator at 37 °C. Five days after culture, 30 µM of 5-fluoro-2'-deoxyuridine (Cat#: F0503, Sigma Aldrich) was treated to inhibit the growth of glial cells. All procedures for experiments using mice were approved and followed according to the guidelines of the Johns Hopkins University Animal Care and Use Committee.

### 14. Cell Viability and Cytotoxicity Assay

For cell survival and cytotoxicity analysis, primary cortical neurons were cultured on poly-D-lysine-coated glass coverslips at a density of 10,000 cells/cm², and incubated in a 7 % CO₂ incubator while the medium was replaced twice a week. The cytotoxicity of primary cultured neurons was measured using a LDH cytotoxicity assay kit (Cat#: 88954, Pierce) after 10 DIV mouse cortical neurons were treated with α-syn PFFs (1 µg/ml) for 7 days in the absence or presence of graphene quantum dots (1 µg/ml). In addition, cell death was determined using a TUNEL assay kit (Cat#: 12156792910, Roche). The viability of the primary cultured neurons was measured using an alamarBlue cell viability assay kit (Cat#: DAL1025, Molecular ProbesTM) and a neurite outgrowth staining kit (Cat#: A15001, Molecular ProbesTM). The neurite outgrowth staining kit includes an orange dye for staining the cell membrane surface as a marker for neurite outgrowth, and a cell-permeable dye that is converted from a non-fluorescent matrix to a green fluorescent product by intracellular esterases.

### 15. In Vitro Immunofluorescence

Mouse primary cortical neurons were plated onto poly-D-lysine-coated glass coverslips at a density of 20,000 cells/cm². The neurons were fixed with 4% PFA, and then blocked for 1 hour at room temperature with 5% normal donkey serum (Cat#: 017-000-121, Jackson ImmunoResearch), 2% bovine serum albumin (Cat#: A7030, Sigma Aldrich), and 0.1% Trion X-100 (Cat#: T8787, Sigma Aldrich). The neurons were incubated along with anti-8-OHG (Cat#: ab62623, 1:1,000, Abcam), anti-pS129-α-syn (Cat#: ab59264, 1:1,000, Abcam), and anti-MAP2 (Cat#: MAB3418, 1:1,000, Millipore) antibodies overnight at 4 °C. Samples were washed with PBS containing 0.1% Trion X-100, and then incubated with coverslips in a mixture of FITC-conjugated secondary antibodies (Donkey anti-mouse FITC; Cat#: 715-095-151, Donkey anti-rabbit FITC; Cat#: 711-095-152, Jackson ImmunoResearch) and Cy3-conjugated secondary antibodies (donkey anti-mouse FITC; Cat#: 715-165-151, donkey anti-rabbit CY3; Cat#: 711-165-1527, Jackson ImmunoResearch) at room temperature for 1 hour. Fluorescence images were acquired through a Zeiss confocal microscope (LSM 710, Zeiss Confocla).

### 16. Complex I Activity Assay

Mitochondrial complex I enzyme activity was measured using a complex I enzyme activity microplate assay kit (Cat#: ab 109721, Abcam) in accordance with the manufacturer's instructions. Briefly, primary cortical neurons were plated onto poly-D-lysine-coated 6-cm culture dishes at a density of 1,000,000 cells/dish. The neurons were treated with 1 µg/ml of α-syn PFFs in the presence or absence of 1 µg/ml of graphene quantum dots on day 10 *in vitro.* After 7-day treatment, proteins were extracted from primary neurons using 1/10 volume detergent in PBS, and incubated on ice for 30 minutes. The final protein concentration of each sample was adjusted to 5.5 mg/ml, followed by centrifugation at 12,000 x g for 20 minutes, and the supernatant was loaded in microplate wells and incubated at room temperature for 3 hours. After 3 hours, the plate was washed twice with a buffer solution and 200 µl of an assay solution was added. Mitochondrial complex I enzyme activity was measured for 30 minutes at about 1 minute intervals at OD450.

### 17. Mitochondrial Morphology Assessment

Primary cortical neurons were plated at a density of 10,000 cells/cm² onto poly-D-lysine-coated glass coverslips. The primary cultured cortical neurons were treated with 1 µg/ml of α-syn PFF in the presence or absence of 1 µg/ml of graphene quantum dots on DIV 10. After 7-day treatment, neurons were stained with MitoTracker Orange CMTMRos probes (Cat#: M7510, Life Technologies) according to the manufacturer's instructions. Briefly, primary neurons were incubated with 100 nM MitoTracker® Orange CMTMRos probes for 30 minutes, and then washed with a cell imaging solution (Cat#: A1429DJ, Life Technologies). Stained mitochondria were imaged using a Zeiss confocal microscope (LSM710), and mitochondrial morphological features such as length or aspect ratio (AP, ratio of the major and minor axes of an ellipse equivalent to mitochondria) were analyzed using ImageJ software.

### 18. Determination of Oxygen Consumption Rate

Oxygen Consumption Rate (OCR) was measured by modifying the manufacturer's instructions as follows using the Seahorse XF cell mito stress test kit (Cat#: 103015, Agilent). Briefly, primary cultured cortical neurons were plated onto a hippocampal XF24 cell culture plate at a density of 500,000 cells/well. Neurons were treated with 1 µg/ml of α-syn PFFs with or without 1 µg/ml of graphene quantum dots on DIV 10. After treatment for 7 days, the neurons were washed with warm PBS and incubated in a hippocampal assay medium at 37 °C for 1 hour. Then, the plate was loaded in an XF24 Extracellular Flux Analyzer (Seahorse Bioscience) and the oxygen consumption rate was measured. The oxygen consumption rate was measured at 37 °C with a 1-min mix, 1-min wait, and 2-min measurement protocol, followed by incubation for 45 minutes in a CO₂-free incubator, and analyzed with an XF24 analyzer. Oligomycin, carbonyl kanide m-chlorophenylhydrazone (CCCP), and rotenone were sequentially injected to evaluate basal respiration, coupling of the respiratory chain, and mitochondrial respiration capacity. The measured oxygen consumption rate was normalized using the protein concentration in each well. The data was compared with that of a control and the change was expressed as a percentage.

### 19. Live Imaging

For low-speed confocal live imaging, primary cortical neurons were plated at 10,000 cells on a poly-D-lysine-coated glass bottom dish (Cat#: 150682, NuncTM), and incubated in a 7% CO₂ incubator at 37 °C. On DIV 7, primary cultured neurons were cultured for 1 hour with FITC-labeled α-syn PFFs, 1 µg/ml of GQDs-biotin and a streptavidin Qdot complex, and 100 nM LysoTrackerTM Blue DND-22 (Cat#: L7525, Life Technologies) containing a cell imaging solution for 1 hour. The culture dish was mounted on a Zeiss confocal microscope (LSM710) equipped with a temperature-controlled CO₂ culture system, and then slow-speed images were captured at predetermined intervals with 488 nm and 561 nm laser excitation.

### 20. Microfluidic Chamber

As a triple-chamber microfluidic device, Xona Microfluidic (Cat#: TCND1000) was used. Glass coverslips were coated with poly-D-lysine before being attached to the microfluidic device. About 100,000 neurons per chamber were plated, chamber 1 (C1) or chamber 2 (C2) was pretreated with 0.5 µg of GQDs on DIV 7, and then chamber 1 (C1) was treated with 0.5 µg of α-syn PFFs. Treatment of the first chamber with α-syn PFFs was performed in all experimental groups to create suitable transfer conditions for the next chamber. A 50 µl-difference of medium volume was maintained between three compartments to control the direction of fluid flow. Neurons were fixed using PBS containing 4% PFA at 14 days after treatment with α-syn PFFs. The fixed neurons in the chamber were incubated for 1 hour at room temperature using a blocking solution containing 5% normal donkey serum, 2% bovine serum albumin, and 0.1% Triton X-100. Neurons were incubated with anti-pS129-α-syn (Cat#: ab59264, 1:1,000, Abcam) and anti-MAP2 (Cat#: MAB3418, 1:1,000, Millipore) antibodies overnight at 4 °C, and the chambers were washed using 0.1% Triton X-100-containing PBS, followed by incubation for 1 hour at room temperature with a mixture of FITC-conjugated secondary antibodies (Jackson ImmunoResearch) and Cy3-conjugated secondary antibodies. Fluorescence images were acquired through a Zeiss confocal microscope.

### 21. Animals

All experimental procedures were performed in accordance with the guidelines of the Laboratory Animal Manual of the NIH Guide to the Care and Use of Animals, approved by the Animal Care and Use committee of the Johns Hopkins Medical Institute. Human α-syn-A53T transgenic mice (B6.Cg-Tg, Prnp-SNCA^{∗} A53T; 23 Mkle/J, stock#: 006823) were purchased from Jackson Lab.

### 22. In Vitro Blood-Brain Barrier Permeability of Graphene Quantum Dots

Primary cultured mouse astrocytes for *in-vitro* blood-brain barrier (BBB) permeability experiments were prepared using 10 C58BL/6 mice. Briefly, primary cultured mixed glial cells (neuroglia) were prepared using 1-d-old C57BL/6 mice. The meninges were removed from the isolated cerebral cortex, and the cerebral cortex was disrupted by a 30-ml syringe with a 19-gauge, 0.5-inch needle. Subsequently, the cells were plated into 75 cm² T-flasks with DMEM supplemented with 10% FBS, 1 mM HEPES, 2 mM glutamine, and antibiotics/antibacterial agents (Life Technologies). The mixed glial cells were maintained in a 5% CO₂ incubator at 37 °C, and the culture medium was replaced twice a week. After 2 weeks, pure astrocytes were isolated using an astrocyte isolation kit (Cat#: 130-096-053, Miltenyl Biotec). Primary brain microvascular endothelial cells (BMECs) of C57BL/6 mice were purchased from Cell Biologics. High purity (>95%) astrocytes and BMECs were monitored by staining the cell-specific markers GFAP (for astrocytes) and CD31 (for BMEC, Cat#: ab28364, 1: 500, Abcam). For the formation of the *in vitro* BBB, 10⁶ cells/ml of astrocytes were added to the underside of collagen-coated 0.4 µm Transwell inserts (Cat#: CLS3491, Sigma Aldrich), and incubated for 48 hours under conditions of 37 °C and 5% CO₂. Subsequently, chambers were carefully placed into a 6-well plate where 10⁷ cells/ml of BMECs were plated on the inserts. To confirm the structural integrity of the *in vitro* BBB, transepithelial electrical resistance was measured using an epithelial volt/ohm (TEER) meter (Cat#: 300523, EVOM2, World Precision Instruments) on day 0, day 2, day 4, day 6, and day 8 after BMEC seeding. In order to confirm the integrity, BBB impermeable 3 kDa dextran-fluorescein (Cat#: D3306, Life Technologies) was loaded inside (the blood side) of Transwells, and the ratio was measured using a fluorescence spectrophotometer (Ex=494 nm/Em=521 nm for dextran-fluorescein; Ex=555 nm/Em=580 nm for BBB impermeable dextran rhodamine). The concentrations of remaining GQDs, biotin-GQDs, nano-GOs, and rGQDs were measured at 520 nm (Ex=310 nm) using FluoroBriteTM DMEM media (Cat#: A1896701, Life Technologies) of inside (the blood side) and outside (the cell membrance).

### 23. Exosome Isolation

To measure the concentration of GQDs-biotin from the released exosomes, BMECs or astrocytes were plated on 6-cm culture dishes, and 5 µg of GQDs-biotin were treated for 12 hours. After 12 hours, the culture medium was changed, and the exosomes were isolated using an exosome isolation reagent (Cat#: 4478359, ThermoFisher) after 24 and 48 hours.

### 24. In-vivo BBB Permeability of GQDs

For *in vivo* immunostaining of GQDs-biotin, 8-week-old C57BL/6 mice were intraperitoneally injected with 2 mg/kg GQDs-biotin. The brain was extracted, fixed with 4% PFA for 6 hours, dehydrated in 30% sucrose for 48 hours, and subjected to immunohistochemical analysis. The GQDs-biotin signals of the olfactory bulb, neocortex, midbrain, and cerebellum were visualized using a DAB kit. The GQDs-biotin positive signals in cells of the aforementioned regions were confirmed by immune-EM staining using a GoldEnhanceTMEM Plus solution with 20-min incubation, according to the manufacturer's instructions. For *in vivo* BBB experiments, 8-week-old C57BL/6 mice were injected with biotin (2 mg/kg) or a vehicle intraperitoneally or intravenously. On days 7 and 14, the brain and blood were harvested, and the brain was homogenized with 1% TX-100 in PBS. The blood was maintained at room temperature for 30 minutes to coagulate whole blood, and the clot was removed by centrifugation at 2,000 x g for 10 minutes. The concentration of GQDs-biotin was measured using a QuantTag biotin quantification kit (Cat#: BDK-2000, Vector Laboratories). The concentration ratio of brain/plasma of GQDs-biotin was calculated for the brain/plasma ratio.

### 25. Stereological Assessment of Dopamine Neuronal Death

All experimental procedures were performed in accordance with the guidelines of the Laboratory Animal Manual of the NIH Guide to the Care and Use of Animals, approved by the Animal Care and Use committee of the Johns Hopkins Medical Institute. 8- to 10-week-old male C57BL6 mice were purchased from the Jackson Laboratory. The mice were anesthetized with pentobarbital (60 mg/kg) and 2 µl of PBS or PFFs (5 µg/2 µl) was injected into one hemisphere of the striatum of each mouse by using a stereotaxic instrument (Cat#: Model 900; David KOPF instruments). 50 µl of GQDs (50 µg per mouse) was intraperitoneally injected into experimental groups biweekly for 6 months. After 6 months of injection, the brain of mice was perfused with PBS and 4% PFA. After the brain was fixed with 4% PFA for 12 hours, the brain was dehydrated with 30% sucrose and analyzed by immunohistochemistry. The whole brain including the substantia nigra (SN) was cut into 50 µm coronal sections, and every fourth section was used for the analysis of the number of dead cells. The sections were incubated with rabbit polyclonal anti-TH (Cat#: NB300-19; 1:1,000; Novus Biologicals) or rabbit polyclonal anti-pS 129-α-syn (Cat#: ab59264; 1:1,000; Abcam) with a blocking solution. After visualization using a DAB kit (Cat#: SK-4100; Vector Laboratories), the sections were incubated with a biotinylated secondary antibody and streptavidin-conjugated horseradish peroxidase (HRP) (Cat#: PK-6101; Vector Laboratories). Stained tissue sections were loaded on slides, and then the Nissl body was stained with thionin. The total number of TH-and Nissl-positive neurons in the SN was measured using the Optical Factionator probe program of Stereo Investigator software (MBF Bioscience).

### 26. In-vivo Glial Cell Immunohistochemistry

Animal brains were perfused with PBS and 4% PFA. After post-fixation and freezing, sections were made and incubated with anti-Iba-1 antibodies (Cat#: 019-19741; 1:1,000; Wako) or anti-GFAP antibodies (Cat#: Z0334; 1:2,000; Dak). The cultured tissue was cultured again with biotin-conjugated anti-rabbit antibodies and ABC reagent (Cat#: PK-6101; Vector Laboratories), and then each section was stained with a DAB peroxidase substrate (Cat#: SK-4100; Vector Laboratories). The number and density of glial cells in the SN were measured using ImageJ software (http://rsb.info.nih.gov/ij/, NIH). For histopathology of major organs, 8- to 10-week-old C57BL/6 mice were intraperitoneally injected with 50 µg of GQDs biweekly for 6 months. After 6 months of injection, animals were perfused and fixed using PBS and 4% PFA. The liver, kidney, and spleen were extracted and stained with an H&E stain kit (Cat#: H-3502; Vector Laboratories).

### 27. α-syn Aggregation Formation Assay

HEK293T cells were plated on glass slides and transfected with a pCMV5 vector with a myc-tagged A53T α-syn mutant (pCMV5-myc-SNCA-A53T, kindly gifted by Dr. Thomas C. Sudof), and then treated with PBS (pH 7.4) or GQDs (0.1 µg/ml). 48 hours after the treatment, HEK293T cells were washed three times with PBS and fixed at room temperature for 20 minutes using PBS containing 4% PFA. After washing three times with PBS, the fixed cells were washed for 4 minutes using PBS containing 0.1% Triton X-100 (Sigma Aldrich). Thereafter, the cells were washed three more times with PBS and blocked with PBS containing 5% donkey serum for 20 minutes. α-syn expression was monitored using an α-syn antibody (Cat#: 610787; 1:1,000; BD Biosciences). Signals appearing at 550 nm and 570 nm were imaged by a laser scanning confocal microscope. The number of immune positive aggregates per field was measured, quantified using ImageJ software (http://rsb.info.nih.gov/ij/, NIH), and normalized using the number of cells stained with DAPI.

### 28. Behavioral Analyses

### (1) Cylinder Test

For the test, asymmetry in forelimb use was used. Experimental animals were placed in a 20-cm clear plastic cylinder and the number of times of contact of a forepaw with the wall of the cylinder was measured. About 20 to 30 wall touches per animal (when anterior/posterior contacts and contacts opposite thereto were fully performed) were counted. The number of forelimb contacts corresponding to the injured cerebrum was divided by a total number of forelimb contacts and expressed as a percentage. All analyzes were performed by blinded investigators for different groups.

### (2) Pole Test

All animals were acclimated for 30 minutes prior to behavioral experiments. A behavioral test pole was made using a 75-cm metal pole having a diameter of 9 mm which was wrapped with bandage gauze. Each mouse was placed head-up at the top of the pole (7.5 cm from the top of the pole), and the total time taken to reach the bottom of the pole was recorded. All mice were trained for two successive days before the actual trial. Each training session consisted of three individual training sessions, and on the test day, all mice were evaluated with three sessions and the total time was recorded. The maximum cutoff time to stop testing and recording was 60 seconds. Results for the turn down, climb down, and the total time (in seconds) were recorded.

### (3) Clasping Test

For hA53T α-syn mice, hindlimb clasping was tested. The hindlimb clasping of animals lifted by their tails was observed for 10 seconds, and scores for each test were determined based on the following criteria.
1) Score 0: When hindlimbs are spread outwards and away from the abdomen.
2) Score 1: When single hindlimb is drawn back towards the abdomen for five seconds or longer.
3) Score 2: When both hindlimbs are partially drawn back towards the abdomen for five seconds or longer.
4) Score 3: When both hindlimbs are completely drawn back towards the abdomen for five seconds or longer.

### 29. Statistics

Date was expressed as mean ± standard deviation from at least three independent experiments. To evaluate the statistical significance, a Student t test or ANOVA test was performed with Bonferroni post-analysis using Prism6 software (GraphPad). Evaluations with p <0.05 were considered significant.

### Experimental Results

### 1. Effect of GQDs on α-syn Fibrillization and Fibril Disaggregation

FIG. 1A is a view illustrating effects of α-syn fibrillization and fibril disaggregation according to the presence or absence of GQDs prepared according to the present invention.

FIG. 1B is a graph showing the results of quantifying the degree of α-syn fibrillization using ThT fluorescence results. As illustrated in FIG. 1B, it was confirmed that, when only α-syn was present, α-syn fibrillization rapidly increases and the fluorescence value increases, whereas, when graphene quantum dots are reacted in combination, α-syn fibrils were not formed even after 150 hours, and thus there was no change in fluorescence value.

FIG. 1C is a graph showing the results of turbidity analysis at each reaction time point of 0, 2, 4, 6, 8, 10, 12, 24, 72, and 168 hours (n = 4, ****P<0.001, two-way ANOVA with a post-hoc Bonferroni test, error bars are standard deviation). As illustrated in FIG. 1C, it was confirmed that, when only α-syn was present, α-syn fibrillization increases rapidly and turbidity increases, whereas, when graphene quantum dots are reacted in combination, α-syn fibrils are not formed and thus turbidity did not increase.

FIG. 1D illustrates TEM images after fibrillization in the presence (right) and absence (left) of GQDs. As illustrated in FIG. 1D, it was confirmed that, when no GQDs were present, α-syn fibrillization proceeded, whereas, when GQDs were reacted in combination, α-syn fibrils were not formed even after 7 days.

FIG. 1E is a graph showing the results of disaggregation of α-syn fibrils already formed after incubation with GQDs using samples of reactions monitored by ThT fluorescence. As illustrated FIG. 1E, it was confirmed that, when the α-syn fibrils and GQDs were reacted together, α-syn fibrils were disaggregated and the ThT fluorescence value decreased.

FIG. 1F is a graph showing turbidity analysis results according to various reaction time points (0, 1, 3, 12, 24, 72, and 168 hours, at each time point, n = 4, ****P <0.001, two-way ANOVA with a post-hoc Bonferroni test, error bars are standard deviation). As illustrated in FIG. 1F, it was confirmed that, when α-syn fibrils and GQDs were reacted together, α-syn fibrils were disaggregated and turbidity was reduced.

FIG. 1G is a graph showing end-to-end length (n= 50 fibrils at each time point) and the number of fibrils (n= 4 at each time point) for the α-syn fibril disaggregation effect of GQDs. As illustrated in FIG. 1G, it was confirmed that, after reacting graphene quantum dots and α-syn fibrils for 6 hours, the length of α-syn fibrils was significantly reduced, and after 24 hours, the number of α-syn fibrils was also reduced.

FIG. 1H is a graph showing analysis results showing the amount of remaining α-syn fibrils, determined by multiplying the end-to-end length and number of α-syn fibrils at the same time points (0, 6, 12, 24, and 72 hours) in the presence of GQDs. As illustrated in FIG. 1H, it was confirmed that the amount of α-syn fibrils was significantly reduced by the GQDs.

FIG. 1I illustrates TEM images of α-syn fibrils at various reaction time points (6 hours, 12 hours, 1 day, 3 days, and 7 days) in the absence (top) or presence (bottom) of GQDs. As illustrated in FIG. 1I, it was directly confirmed that the length of α-syn fibrils was reduced in the presence of GQDs, and the formed α-syn fibrils were effectively disaggregated.

FIG. 1J illustrates measurement results of α-syn fibrils by dot-blot analysis at various reaction time points (0 hours, 12 hours, 1 day, 3 days, and 7 days) using α-syn filament-specific antibodies. As illustrated in FIG. 1J, it was confirmed that, as the reaction time increased, the amount of α-syn fibrils was significantly decreased, and α-syn fibrils were not detected on day 7.

FIG. 1K illustrates BN-PAGE analysis results of α-syn prepared as a sample of the reaction at various reaction time points (0 hours, 3 hours, 6 hours, 12 hours, 1 day, 3 days, and 7 days). As illustrated in FIG. 1K, it was confirmed that all α-syn fibrils were disaggregated and present in a monomer state on day 7 after reaction with the GQDs.

Through the above results, it was confirmed that the graphene quantum dots of the present invention not only can inhibit α-syn fibrillization, but can also effectively disaggregate already formed α-syn fibrils.

### 2. Analysis of Interaction between GQDs and Already Formed α-syn Fibrils During Disaggregation

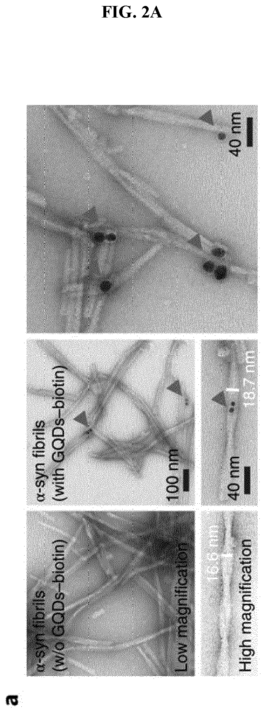

FIG. 2A illustrates TEM results showing the binding between biotinylated GQDs and α-syn fibrils at low and high magnifications. Triangular arrow represent biotinylated GQDs bound to ultra-small gold-streptavidin nanoparticles. As illustrated in Fig. 2A, it was confirmed that GQDs and α-syn fibrils were bound.

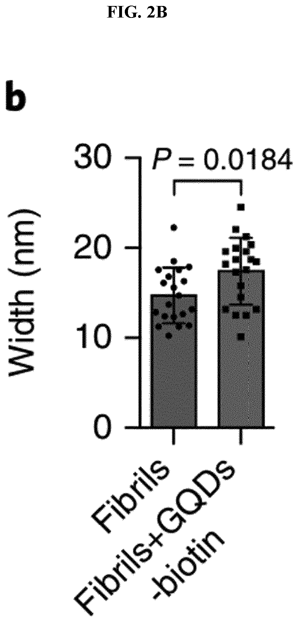

FIG. 2B is a graph showing changes in the average width of α-syn fibrils in the disaggregation process after 1 hour of incubation (n = 20 for each group, *P<0.05, Student's t test, error bars are standard deviation). As illustrated in FIG. 2B, it was confirmed that the GQDs were bound to α-syn fibrils, and thus the average width was increased.

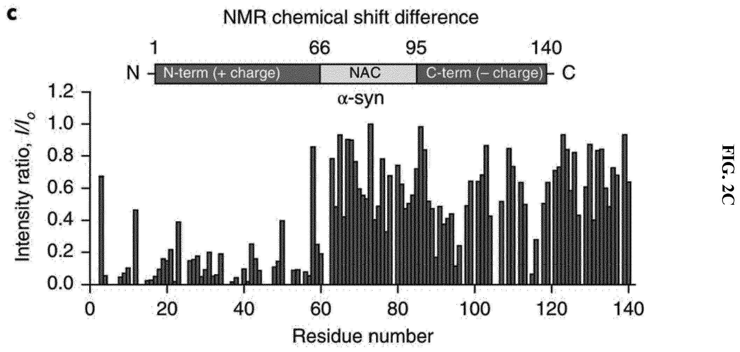

FIG. 2C is a graph showing NMR chemical shift differences obtained from ¹H-¹⁵N HSQC spectra. The decreased intensity ratio of NMR chemical shifts for each residue after binding with GQDs can be confirmed. As illustrated in FIG. 2C, it was confirmed that GQDs were bound to the NAC and C-terminal portions of α-syn fibrils.

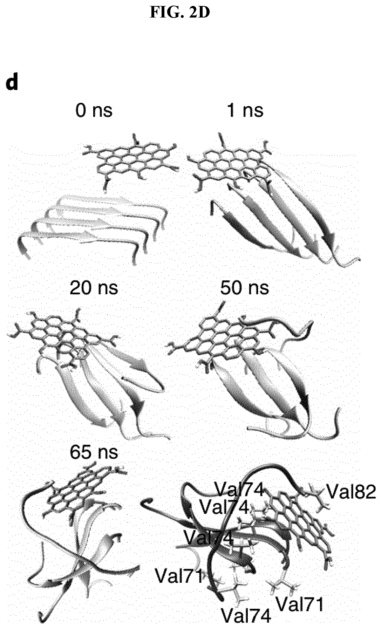

FIG. 2D illustrates 65 ns snapshot images of the interaction of GQDs and already formed α-syn fibrils by using time course simulation dynamics. As illustrated in FIG. 2D, it was predicted that the GQDs change the structure of α-syn fibrils by binding to α-syn fibrils.

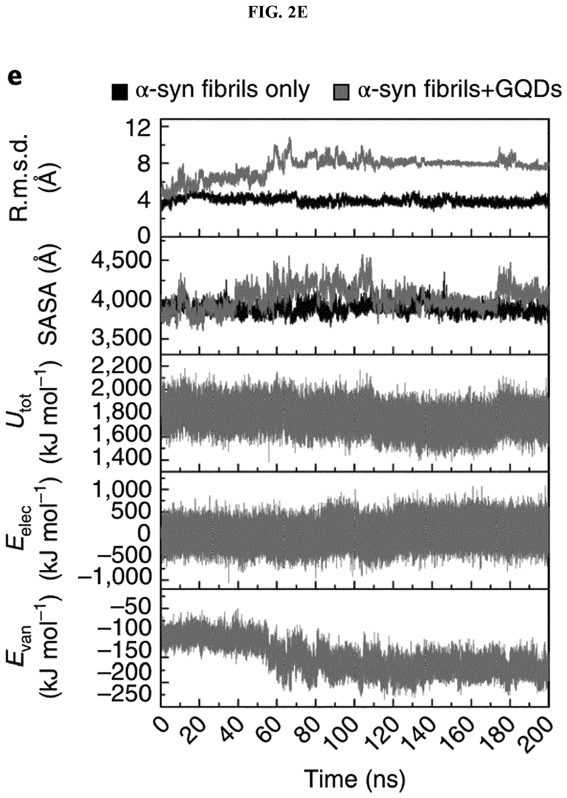

FIG. 2E illustrates results showing time-dependent plots for the RMSD of atomic positions, SASA for an α-syn fibril group (black) and an α-syn fibril and GQD group (red), total potential energy (ΔUₜₒₜ), electrostatic energy (ΔElec), and van der Waals energy (ΔEᵥₐₙ) for the α-syn fibril and GQD group.

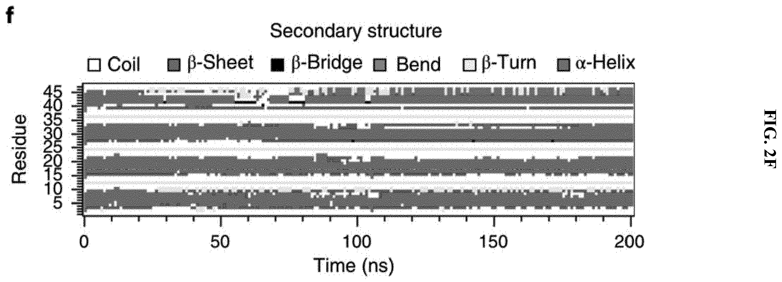

FIG. 2F illustrates a time-dependent plot calculated by the DSSP algorithm. As illustrated in FIG. 2F, it was confirmed that the beta-sheet was reduced and the alpha-helix was increased.

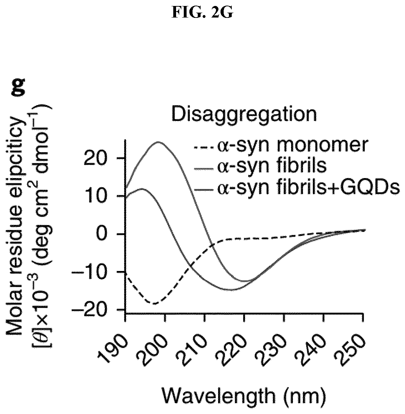

FIG. 2G is a graph showing CD spectra of α-syn monomers and α-syn fibrils without or with GQDs after incubation for 7 days.

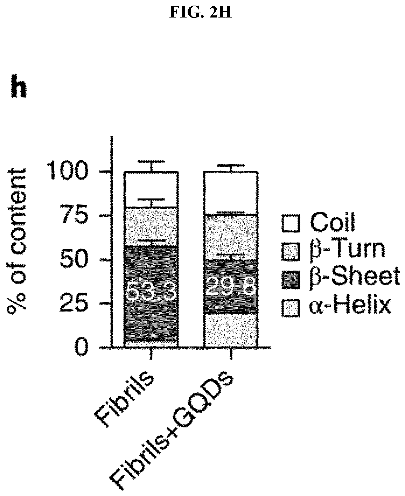

FIG. 2H is a graph showing the content ratio of α-syn fibrils and fractional secondary structures of α-syn fibrils disaggregated by GQDs, calculated using the algorithm CONTIN/LL. As illustrated in FIG. 2H, it was confirmed that the β-sheet was decreased and the α-helix was increased.

Through the above results, it was confirmed that the graphene quantum dots of the present invention can promote the disaggregation of α-syn fibrils by directly binding to the α-syn fibrils to change the structure of the α-syn fibrils.

### 3. Effect of GQDs on α-syn PFF-induced Neuronal Death, Pathology, and Transmission in vitro

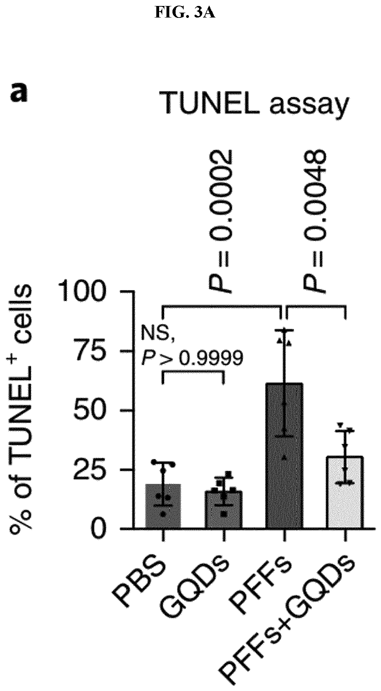

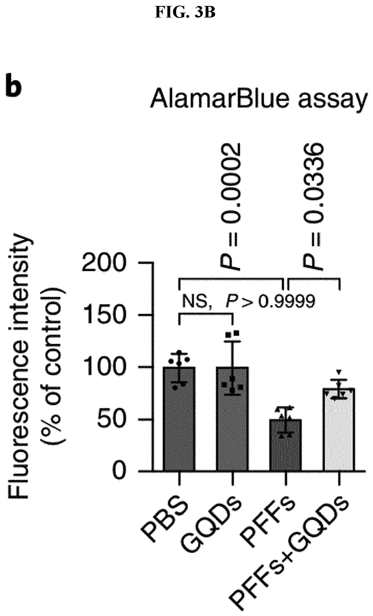

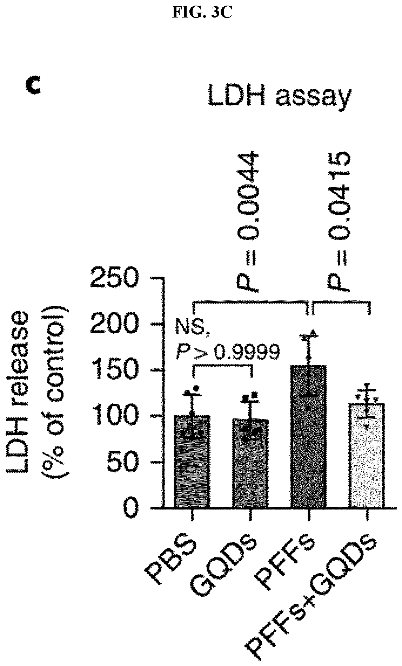

FIGS. 3A to 3C are graphs showing neuronal death assessed by TUNEL (FIG. 3A), alamarBlue (FIG. 3B), and LDH assays (FIG. 3C) after 10 DIV mouse cortical neurons were treated with α-syn PFFs (1 µg/ml) for 7 days in the absence and presence of GQDs (1 µg/ml) (n=4). As illustrated in FIGS. 3A to 3C, it was confirmed that the GQDs did not exhibit cytotoxicity and significantly reduced cytotoxicity caused by α-syn PFFs.

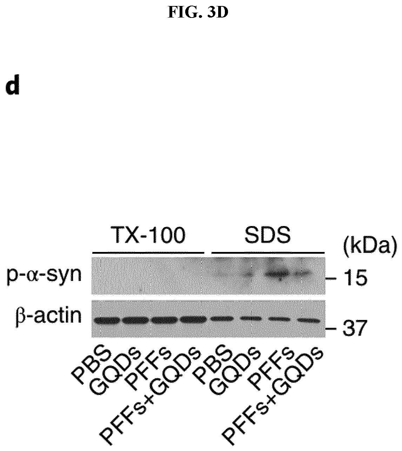

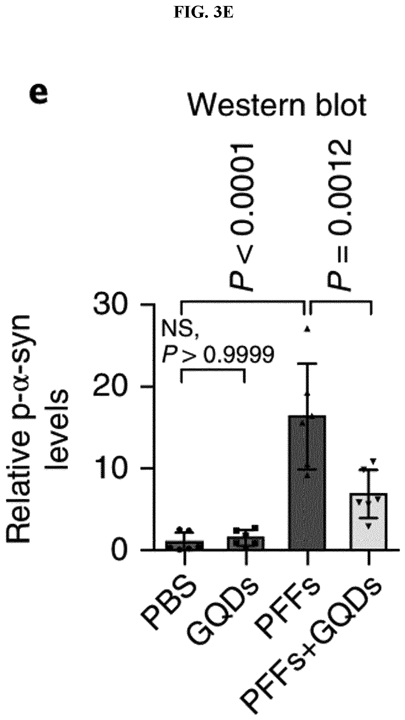

FIG. 3D illustrates images showing immunoblotting results obtained using a p-α-syn antibody, and FIG. 3E is a graph showing the results of quantifying the amount of p-α-syn of the SDS-insoluble fraction normalized to β-actin (n=3). As illustrated in FIGS. 3D and 3E, it was confirmed that, when GQDs were co-treated, α-syn PFFs were remarkably reduced.

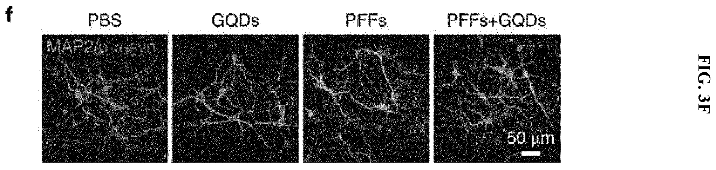

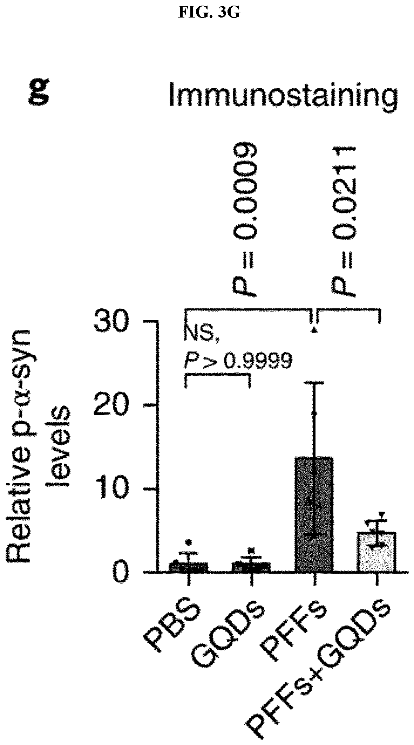

FIG. 3F illustrates immune-microscope images of p-α-syn and a p-α-syn antibody in neurons, and FIG. 3G is a graph showing the results of quantifying the degree of p-α-syn immunofluorescence intensity normalized to a PBS control (n= 3). As illustrated in FIGS. 3F and 3G, it was confirmed that red fluorescence was remarkably increased in the experimental group treated with α-syn PFFs, whereas, when GQDs were co-treated, α-syn PFFs were disaggregated, thus remarkably reducing the red fluorescence value.

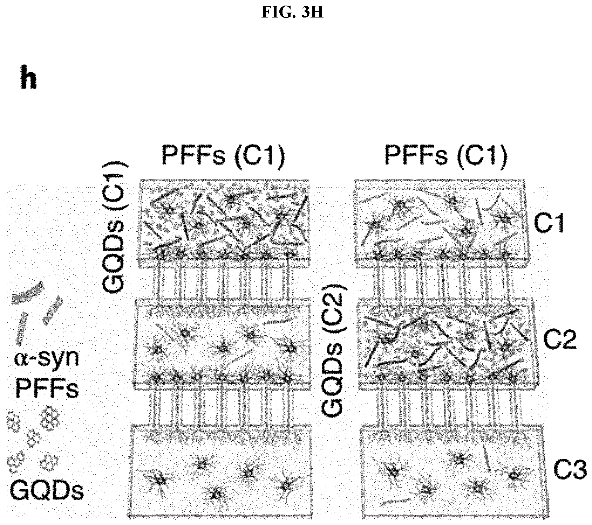

FIG. 3H illustrates a microfluidic device consisting of three chambers for confirming the transmission of pathologic α-syn.

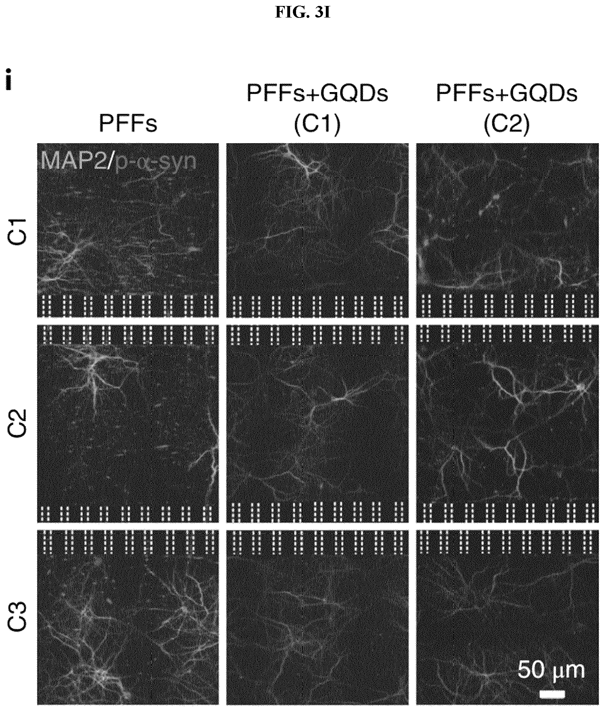

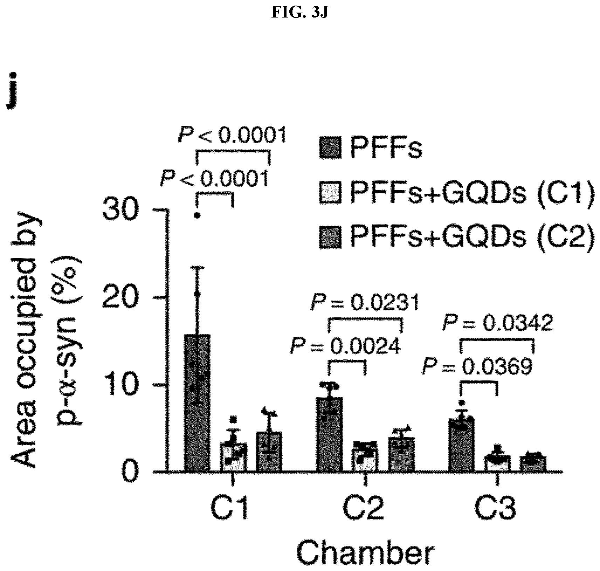

FIG. 3I illustrates representative images of p-α-syn immunostained neurons in the microfluidic device 14 days after α-syn PFFs were added. FIG. 3J is a graph showing quantified p-α-syn immunofluorescence intensities. Areas occupied by p-α-syn were measured in each chamber (n = 3, *P<0.05, **P<0.01, ***P<0.001, NS, two-way ANOVA with post-hoc Bonferroni test, error bars are standard deviation). As illustrated in FIGS. 3I and 3J, it was confirmed that high red fluorescence (a-syn fibrils) was observed in all columns in the case of a control treated with α-syn PFFs in the first column, whereas the amount of α-syn PFFs was remarkably reduced in both an experimental group treated with both GQDs and α-syn PFFs in the first column (C1) and an experimental group treated with α-syn PFFs in the first column (C1) and GQDs in the second column (C2), and thus red fluorescence was hardly observed.

Through the above results, it was confirmed that the graphene quantum dots of the present invention can remarkably inhibit α-syn fibrillization by α-syn PFFs in neurons. It was also confirmed that, as α-syn PFFs are transferred into neurons, graphene quantum dots can also inhibit fibrillization by α-syn PFFs transferred in combination. Through these results, it was confirmed that the graphene quantum dots of the present invention may be effectively used to inhibit and treat interneuronal disease transmission by α-syn fibrillization.

### 4. Effect of GDQs on in vivo α-syn-induced Pathology

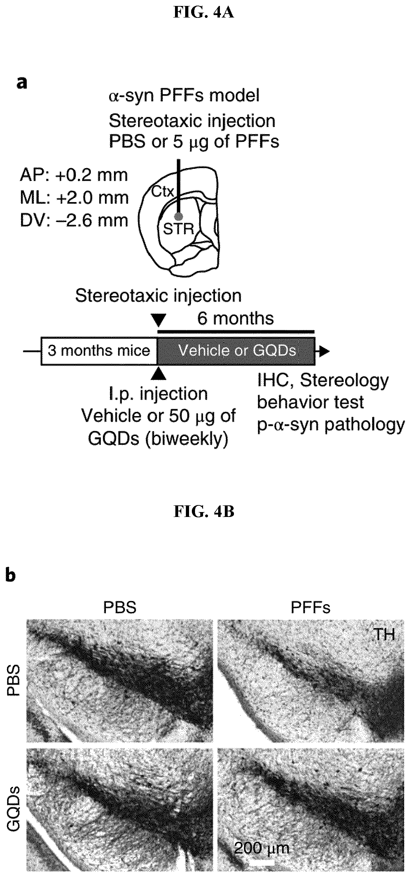

FIG. 4A illustrates the coordinates of α-syn PFFs (5 µg) injected into the striatum of C57BL/6 mice. 50 µg of GQDs or PBS was intraperitoneally injected every other week for 6 months.

FIG. 4B illustrates representative TH immunohistochemistry images in the substantia nigra of an α-syn PFF-injected hemisphere in the absence (top) and presence (bottom) of GQDs. As illustrated in FIG. 4B, it was confirmed that neuronal death was shown in an experimental group administered α-syn PFFs alone, resulting in reduced shading, whereas neuronal death induced by α-syn PFFs was inhibited in an experimental group co-administered GQDs, and thus the degree of shading was recovered to an extent similar to a normal control.

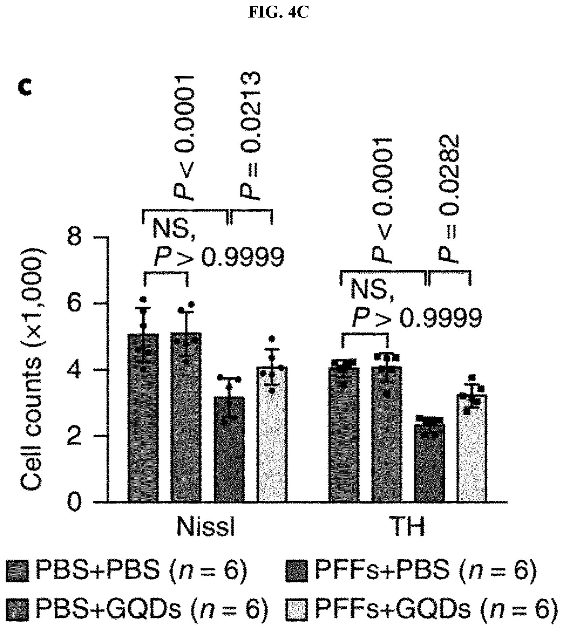

FIG. 4C is a graph showing results obtained by stereologically counting the number of TH- and Nissl-positive neurons in the SN via stereological analysis after 6 months of α-syn PFF injection with and without the injection of GQDs (n= 6 per each group). As illustrated in FIG. 4C, it was confirmed that α-syn PFF-induced neuronal death was reduced in an experimental group administered both α-syn PFFs and GQDs.

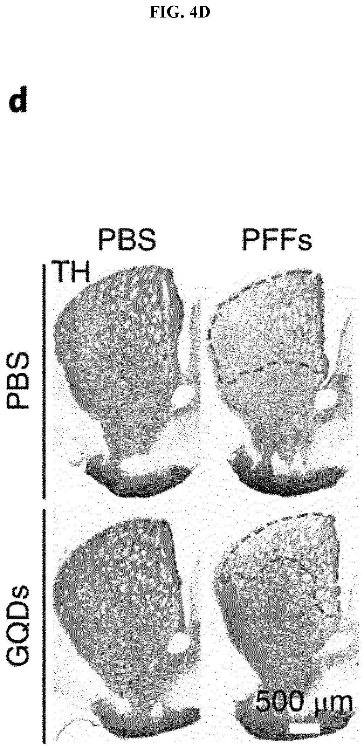

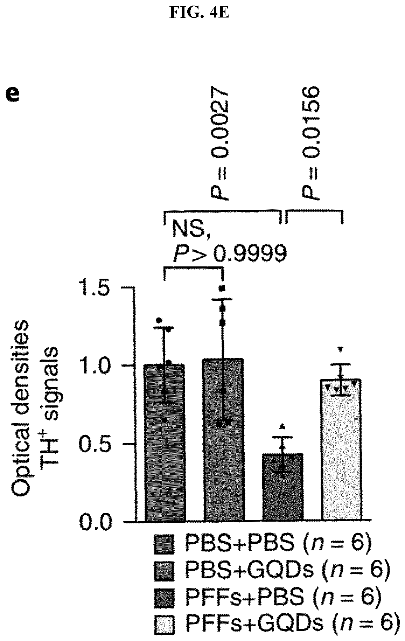

FIG. 4D illustrates representative TH immunohistochemistry images in the striatum of an α-syn PFF-injected hemisphere, and FIG. 4E is a graph showing the results of quantifying the density of TH-immunopositive fibers in the striatum (n= 5 or 6 per group). As illustrated in FIGS. 4D and 4E, it was confirmed that the decrease in the density of TH-immune positive fibers by α-syn PFFs was recovered in the experimental group administered both α-syn PFFs and GQDs.

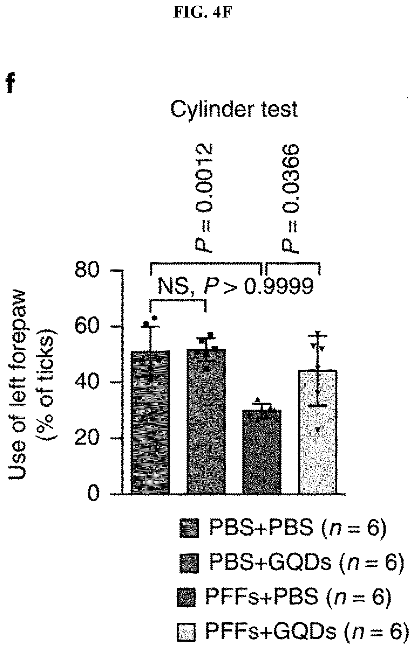

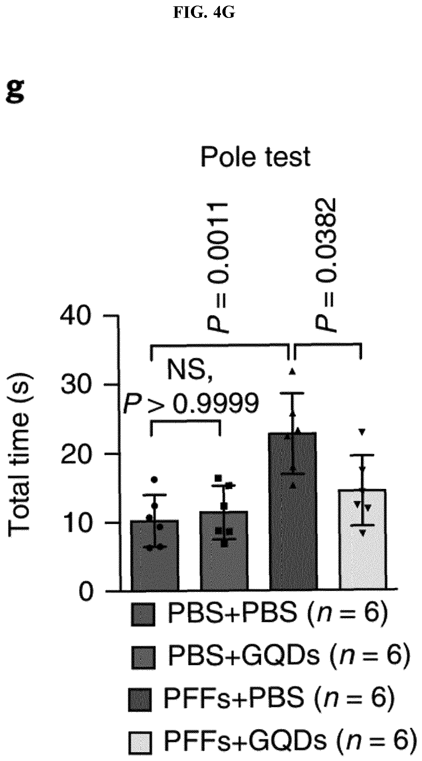

FIG. 4F is a graph showing the results of a cylinder test from among assessments of behavioral deficits measured by the use of forepaws (n= 5 or 6 per group). In addition, FIG. 4G is a graph showing the results of a pole test, which assesses the ability to grasp and recognize a pole, from among assessments of behavioral deficits of mice (n= 5 or 6 per group). As illustrated in FIGS. 4F and 4G, it was confirmed that the abnormal behavior of mice by the administration of α-syn PFFs showed behavioral patterns similar to those of a normal mouse control when GQDs were co-administered.

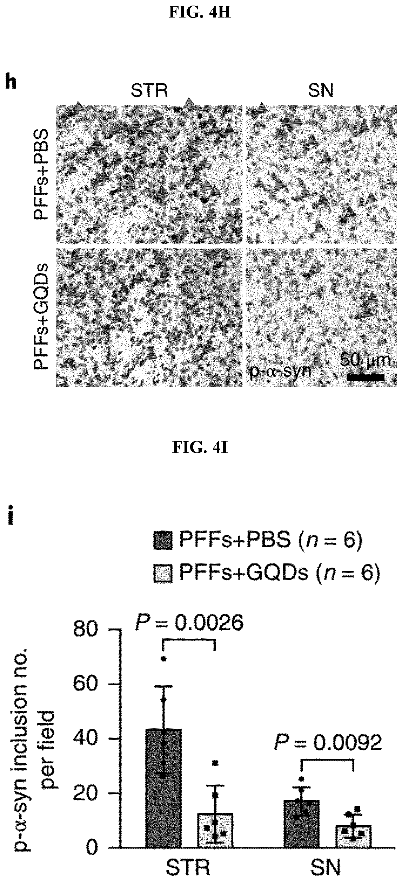

FIG. 4H illustrates representative p-α-syn immunostaining images in the striatum and SN of an α-syn PFF-injected hemisphere, and FIG. 4I is a graph showing quantification results of p-α-syn immunoreactive neurons in the striatum and SN (n= 5). As illustrated in FIGS. 4H and 4I, it was confirmed that the number of α-syn PFF-positive neurons was remarkably reduced in the experimental group co-administered GQDs.

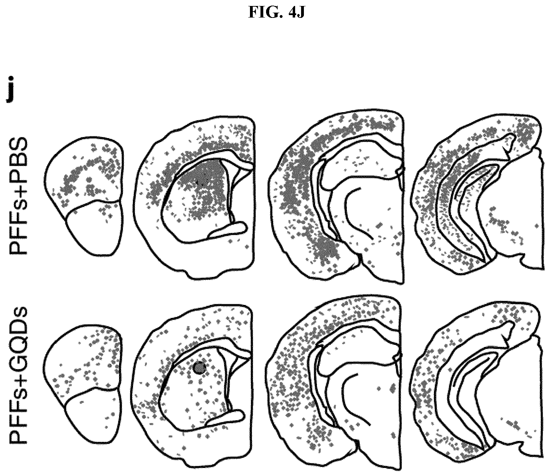

FIG. 4J illustrates the distribution of LB/LN-like pathology in the CNS of an α-syn PFF-injected hemisphere (p-α-syn positive neurons: red dots, p-α-syn positive neurites: red lines). As illustrated in FIG. 4J, it was confirmed that the degree of pathology distribution was remarkably reduced in the experimental group co-administered GQDs.

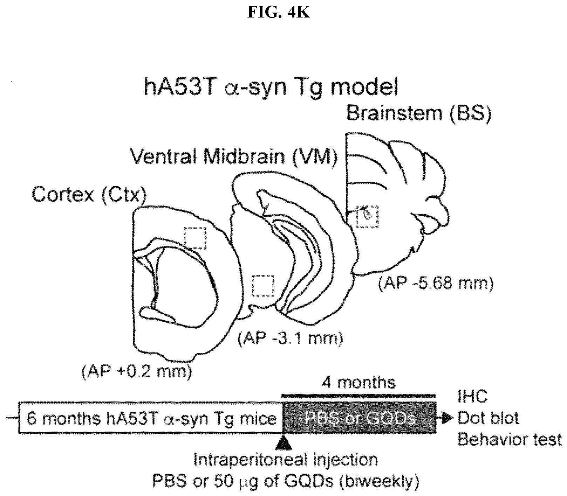

FIG. 4K illustrates the observation areas of a hA53T α-syn Tg model. 50 µg of GQDs or PBS was intraperitoneally injected every other week for 4 months.

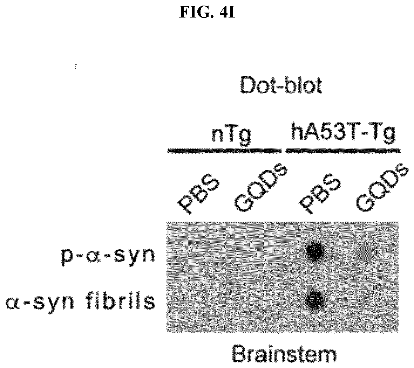

FIG. 4L is an image showing measurement results of p-α-syn and α-syn fibrils by dot-blot assay. As illustrated in FIG. 4L, it was confirmed that p-α-syn and α-syn fibrils were remarkably reduced in the brainstem in the experimental group administered GQDs.

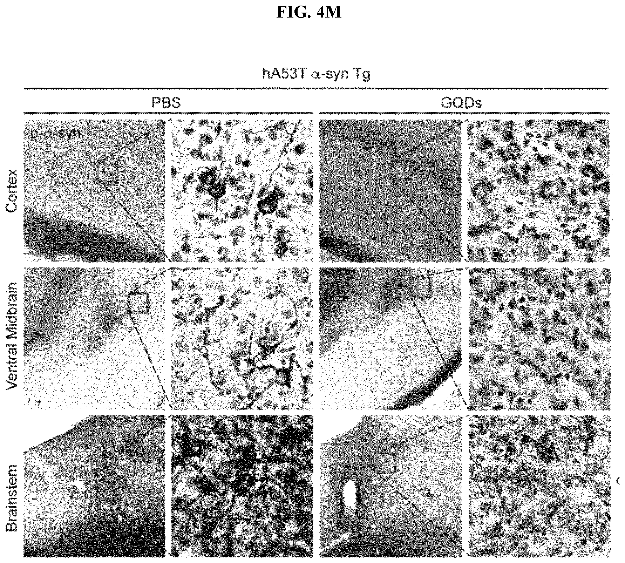

FIG. 4M illustrates representative α-syn immunostaining images in the cortex, ventral midbrain, and brainstem of hA53T α-syn Tg mice (n= 5 per group). As illustrated in FIG. 4M, it was confirmed that the α-syn-aggregated structure was observed in all of the cortex, ventral midbrain, and brainstem of hA53T α-syn Tg mice, whereas the α-syn-aggregated structure was hardly observed in the experimental group administered GQDs.

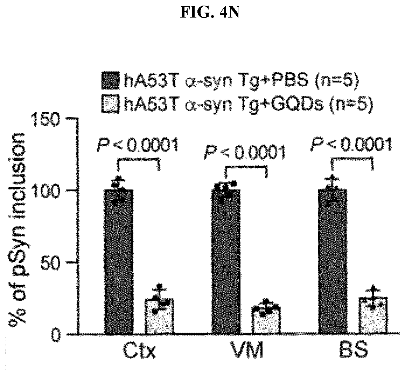

FIG. 4N is a graph showing the results of quantifying (n= 5 per group) p-α-syn immunoreactive neurons in the cortex (Ctx), ventral midbrain (VM), and brainstem (BS) of hA53T. As illustrated in FIG. 4N, it was confirmed that the number of p-α-syn immunoreactive neurons was remarkably reduced in the experimental group administered GQDs.

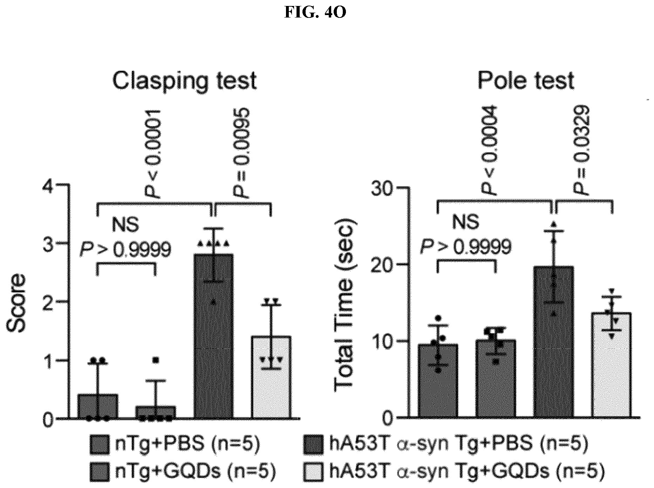

FIG. 4O illustrates a graph (the left side) showing the results of a clasping test from among the assessments of behavioral deficits of mice, and a graph (the right side) showing the results of a pole test from among the assessments of behavioral deficits of mice (n= 5 per group). As illustrated in FIG. 4O, it was confirmed that hA53T α-syn Tg mice had an increased deficiency behavior, whereas the behavioral deficit was recovered in the experimental group administered GQDs.

Through the above results, it was confirmed that the graphene quantum dots of the present invention effectively inhibit the abnormal fibrillization or aggregation of α-syn even *in vivo,* or effectively disaggregate formed α-syn aggregates, and thus can treat pathological symptoms caused by the abnormal fibrillization or aggregation of α-syn.

### 5. Therapeutic effect of GQDs on Etiology of Synucleinopathy

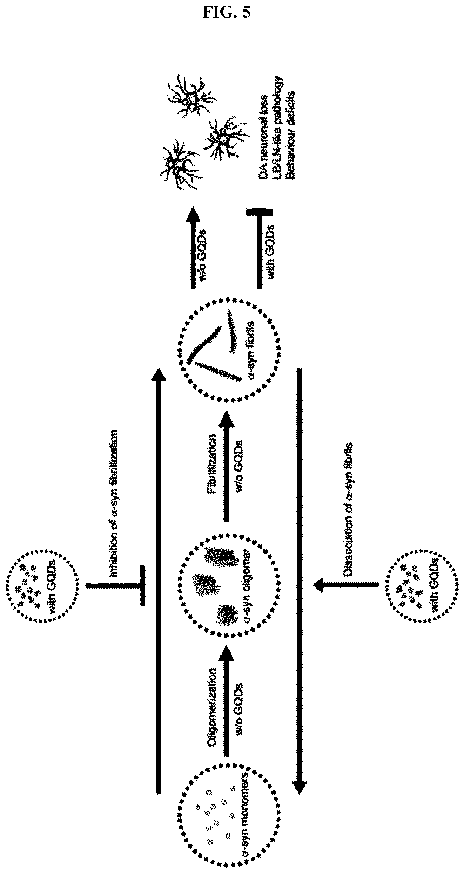

FIG. 5 is a view illustrating the anti-aggregation efficacy of GQDs on the etiology caused by the transmission and propagation of pathological α-syn aggregation *in vitro* and *in vivo.* In the absence of GQDs, pathological α-syn monomers undergo spontaneous fibrillization to form fibrous aggregates, eventually inducing dopaminergic neuron loss, LB/LN analogs, and behavioral abnormalities. In contrast, GQDs inhibit α-syn fibrillization and disaggregate formed fibrils into monomers, and thus can prevent or treat the loss of dopaminergic neurons, and LB/LN analogs and behavioral disorders caused by abnormal α-syn fibrils.

### 6. Synthesis and Biotinylation of GQDs and Analysis of Binding between GQDs-biotin and α-syn Fibrils

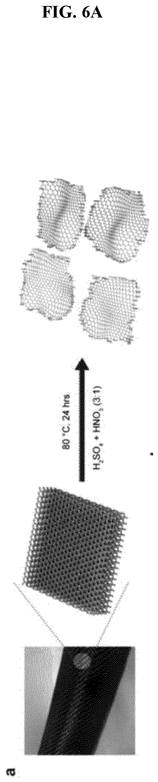

FIG. 6A illustrates GQDs synthesized from carbon fibers by thermo-oxidative cutting.

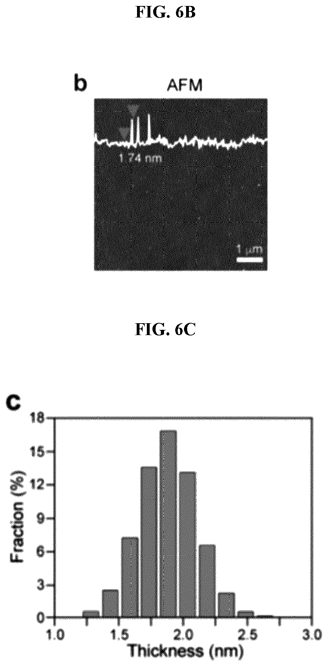

FIG. 6B is an AFM image of synthesized GQDs, and FIG. 6C is a graph showing the thickness distribution of GQDs. As illustrated in FIGS. 6B and 6C, it was confirmed that the average height of GQDs ranged from 0.1 nm to 3 nm.

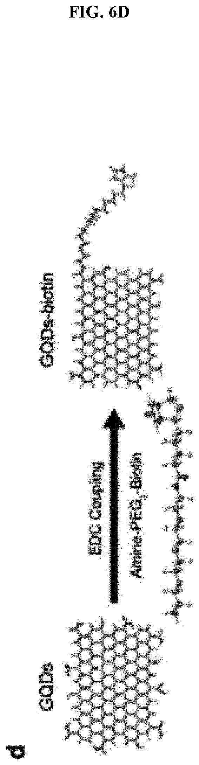

FIG. 6D illustrates a synthesis process of biotinylating GQDs.

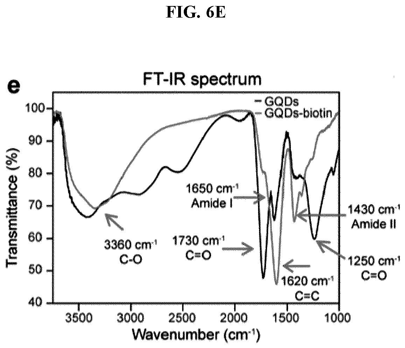

FIG. 6E illustrates the FT-IR spectra of GQDs (black line) and biotinylated GQDs (red line). As illustrated in FIG. 6E, it was confirmed that the biotinylation of GQDs caused a change in functional groups.

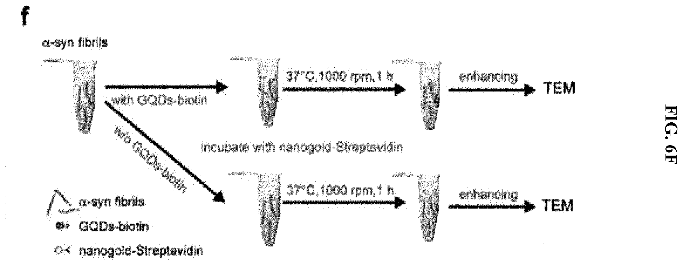

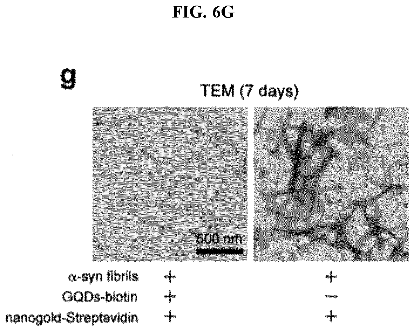

FIG. 6F illustrates a preparation process for the binding affinity between biotinylated GQDs and nanogold-streptavidin, and FIG. 6G illustrates TEM images of α-syn fibrils already formed after 7 days in the presence of a GQD-biotin-nanogold-streptavidin complex (the left side) and a nanogold-streptavidin complex (the right side). As illustrated in FIG. 6G, it was confirmed that the biotinylated GQDs also disaggregated α-syn fibrils by binding to the α-syn fibrils.

### 7. Effect of GQDs on Disaggregation of α-syn Fibrils

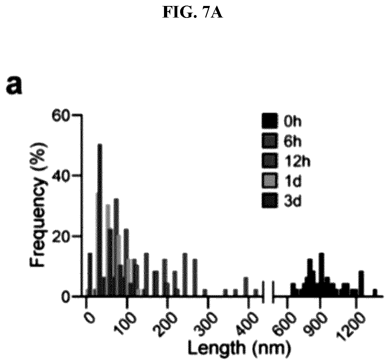

FIG. 7A is a graph showing the length distribution of α-syn fibrils at various reaction time points (0, 6, 12, 24, and 72 hours, n = 50 fibrils at each time point). As illustrated in FIG. 7A, it was confirmed that long α-syn fibrils of 600 nm or more were observed before the reaction, whereas, as the reaction time increased, a monomeric form of α-syn was observed.

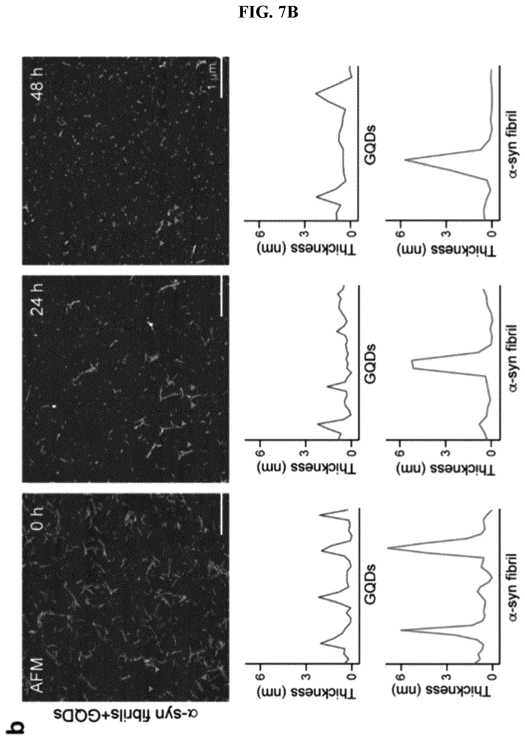

FIG. 7B illustrates AFM images of α-syn fibrils at various reaction time points (0, 24, and 48 hours), showing representative line profiles of designated areas (GQDs: blue lines, α-syn fibrils: red lines). As illustrated in FIG. 7B, it was confirmed that there was no significant difference in height even after reaction between α-syn fibrils and GQDs.

### 8. Effect of GQDs on Disaggregation of α-syn PFFs

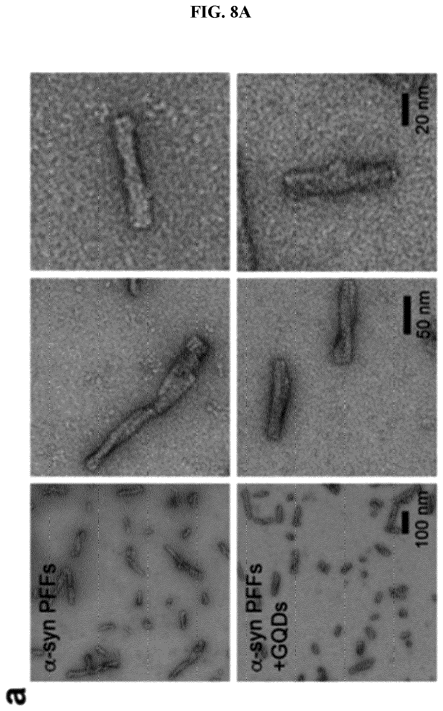

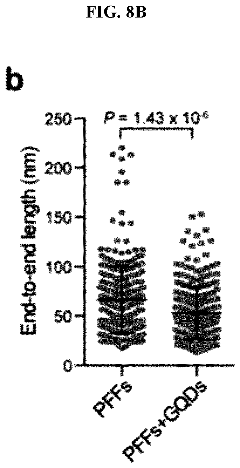

FIG. 8A illustrates representative TEM images of fibrillized α-syn PFFs (5 mg/ml) after reaction for 1 hour in the absence (top) and presence (bottom) of GQDs (5 mg/ml), and FIG. 8B is a graph showing the end-to-end length of α-syn PFFs after reaction for 1 hour (n = 286; PFFs, n = 249; PFFs+GQDs, ***P<0.001; Student's t-test, error bars are standard deviation). As illustrated in FIGS. 8A and 8B, it was confirmed that GQDs disaggregated the fibrillized syn PFFs, thereby reducing the end-to-end length.

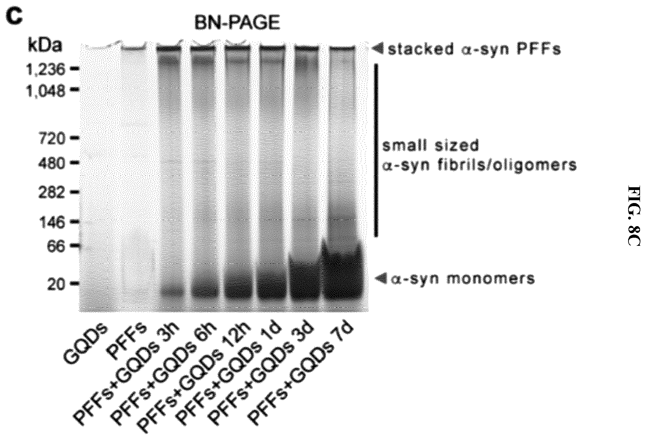

FIG. 8C illustrates the BN-PAGE results of evaluating the amounts of remaining α-syn PFFs and disaggregated α-syn PFFs after various reaction time points. As illustrated in FIG. 8C, it was confirmed that, as the reaction time increased, α-syn PFFs were disaggregated and present in a monomeric form.

### 9. ¹H-¹⁵N HSQC Spectrum Analysis

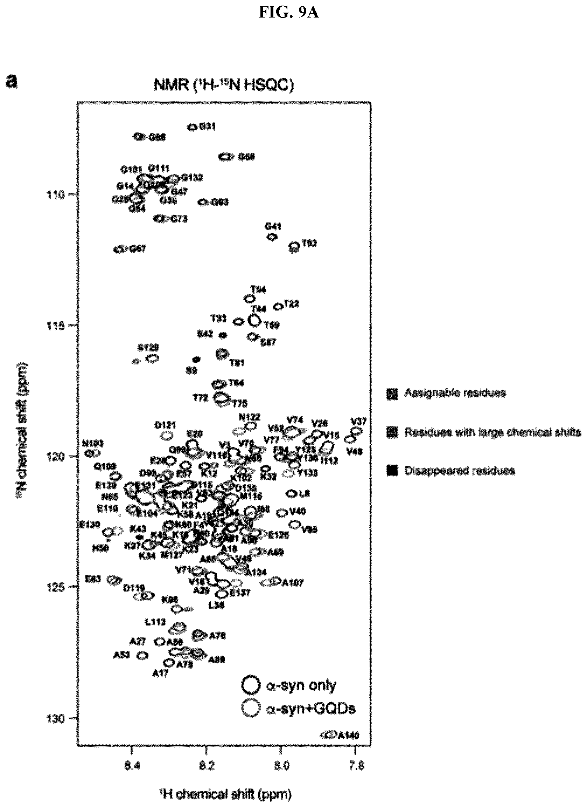

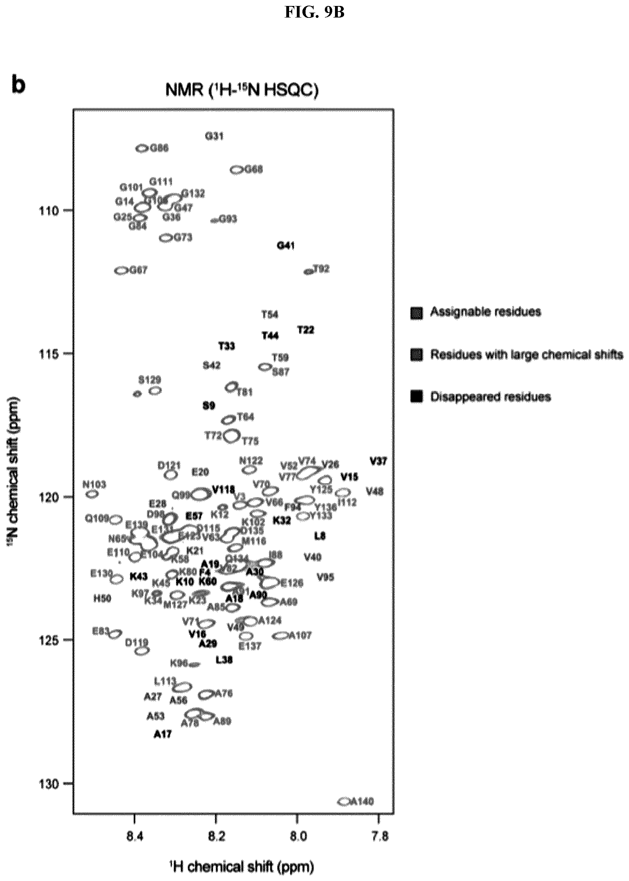

FIG. 9A illustrates full, overlapped ¹H-¹⁵N HSQC spectra of ¹⁵N-labeled α-syn monomers only (black) and after incubation with GQDs (red). The assigned residues appeared only in the ¹⁵N-labeled α-syn monomer group. FIG. 9B illustrates a full HSQC spectrum of ¹⁵N-labeled α-syn monomers incubated with GQDs. Based on the peak intensity of the ¹⁵N-labeled α-syn monomer group, residues with no or minimal decrease in peak intensity (red), residues with a significant decrease in peak intensity (blue), and disappeared residues (black) distinctively appear. Through these results, it was confirmed that the GQDs bind to the N-terminal or positively charged residues of amino acids constituting α-syn.

### 10. Effect of GQDs on α-syn PFF-induced Neuronal Death and Restricted Neurite Outgrowth

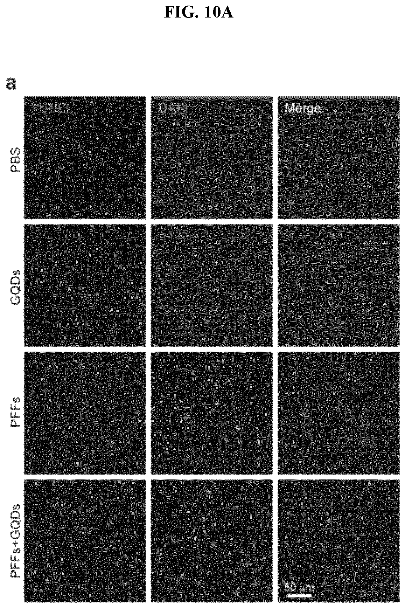

FIG. 10A illustrates representative images of TUNEL-positive neurons. DIV 10 primary cortical neurons were treated with α-syn PFFs (1 µg/ml) in the absence and presence of GQDs (1 µg/ml). TUNEL assay was performed after 7days of incubation. As illustrated in FIG. 10A, it was confirmed that much red fluorescence was observed in the experimental group treated with only α-syn PFFs, whereas red fluorescence was reduced in the experimental group treated with both α-syn PFFs and GQDs, showing a reduction in α-syn PFF-induced cell death.

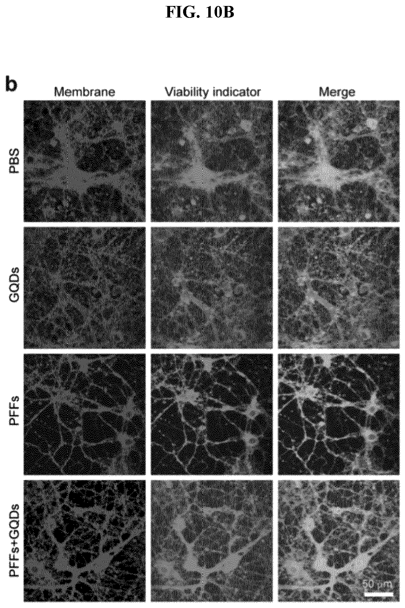

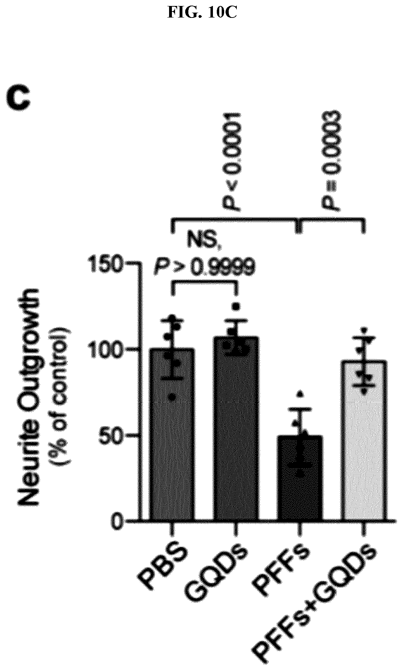

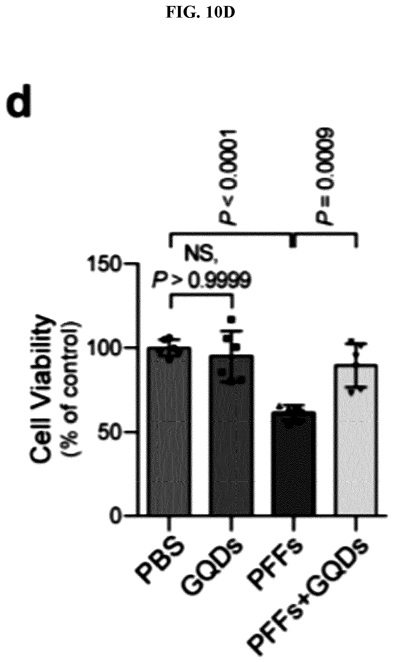

FIG. 10B illustrates representative microscope images showing assays of the outgrowth and cell viability of neurites stained by the outer cell membrane (red) and a cell-permeable viability indicator (green), and FIGS. 10C and 10D are graphs showing the results of quantifying neurite growth and neuron viability. As illustrated in FIGS. 10B to 10D, it was confirmed that both neurite growth and cell viability were reduced in the experimental group treated with only α-syn PFFs, whereas a neurite growth rate similar to that of a control was exhibited in the experimental group treated with both α-syn PFFs and GQDs. It was also confirmed that α-syn PFF-induced cell death was inhibited.

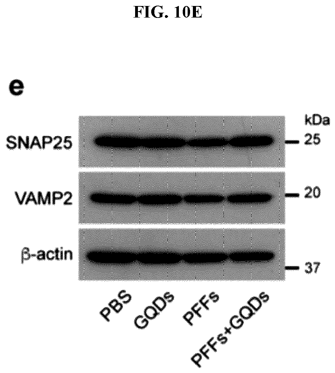

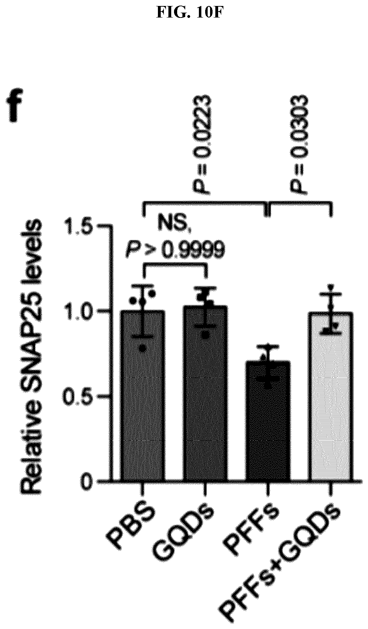

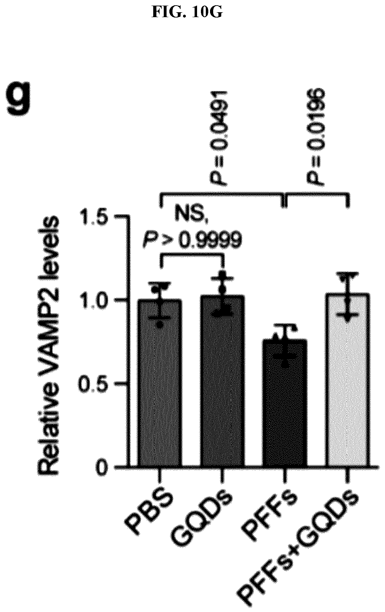

FIG. 10E is a graph showing immunoblot analysis results of SNAP25 and VAMP2 protein levels. 10 DIV primary cortical neurons were treated with α-syn PFFs (5 µg/ml) in the absence and presence of GQDs (5 µg/ml) and incubated for 7 days, followed by immunoblotting. FIG. 10F is a graph showing the results of normalizing the expression levels of SNAP25 to a β-actin expression level and quantifying the normalized levels (n=3), and FIG. 10G is a graph showing the results of normalizing VAMP2 levels to a β-actin expression level and then quantifying the normalized levels (n=3). As illustrated in FIGS. 10E to 10G, it was confirmed that the expression of SNAP25 and VAMP2 proteins was significantly reduced in the experimental group treated with only α-syn PFFs, whereas the SNAP25 and VAMP2 proteins were expressed at a level similar to that of a control in the experimental group treated with both α-syn PFFs and GQDs.

### 11. Effect of GQDs on α-syn PFF-induced Mitochondrial Dysfunction and Oxidative Stress

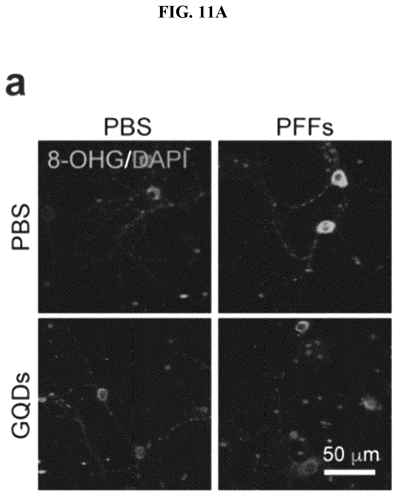

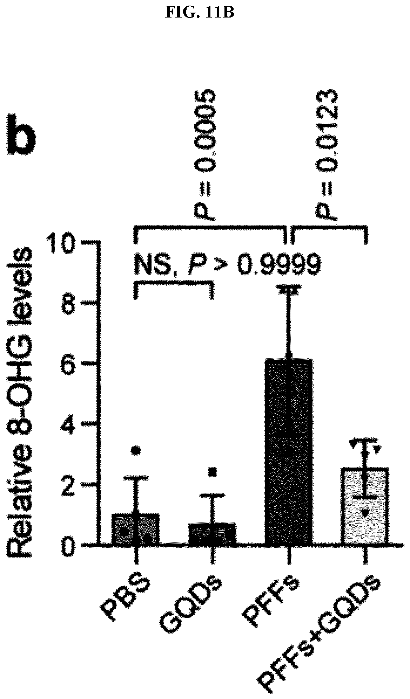

FIG. 11A illustrates representative 8-hydroxyguanine (8-OHG) immunostaining images (n=4 per each group) of primary cortical neurons treated with α-syn PFFs (1 µg/ml) in the absence and presence of GQDs (1 µg/ml) and incubated for 7 days, and FIG. 11B is a graph showing the results of quantifying of 8-OHG content by immunofluorescence levels. As illustrated in FIGS. 11A and 11B, it was confirmed that oxidative stress is increased in the case of being treated with only α-syn PFFs, α-syn PFF-induced oxidative stress was reduced in the case of being treated with both α-syn PFFs and GQDs.

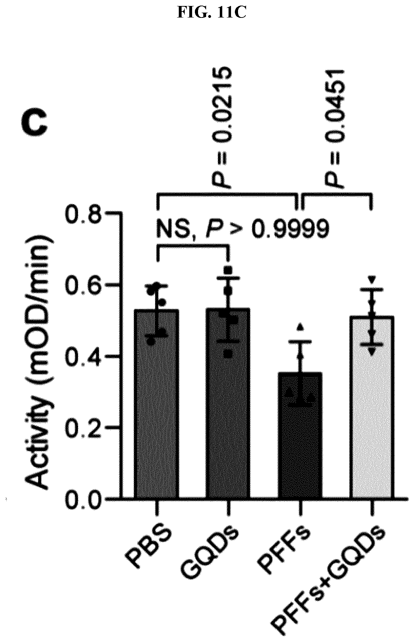

FIG. 11C is a graph showing the results of measuring the activity of a mitochondrial complex after being treated with α-syn PFFs (1 µg/ml) and GQDs (1 µg/ml) and incubated for 7 days. As illustrated in FIG. 11C, it was confirmed that the inhibition of mitochondrial complex activity, induced by α-syn PFFs was recovered by GQDs.

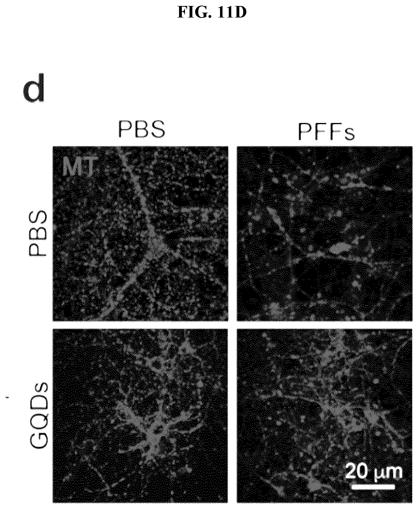

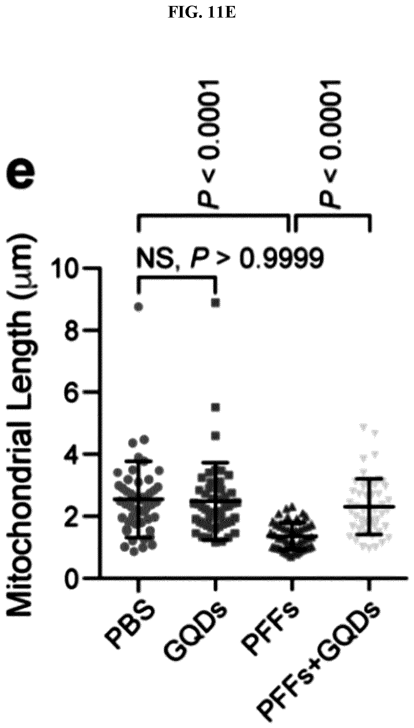

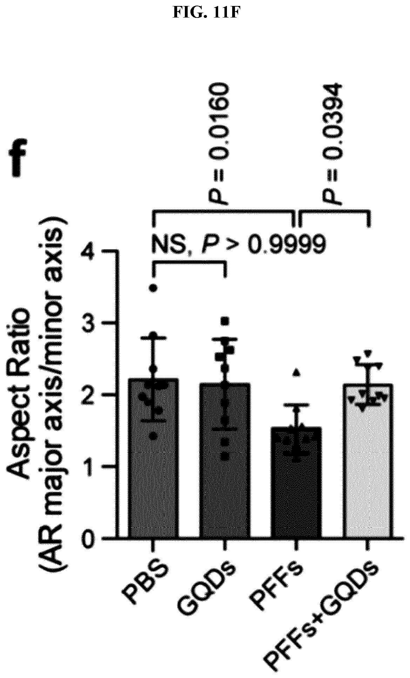

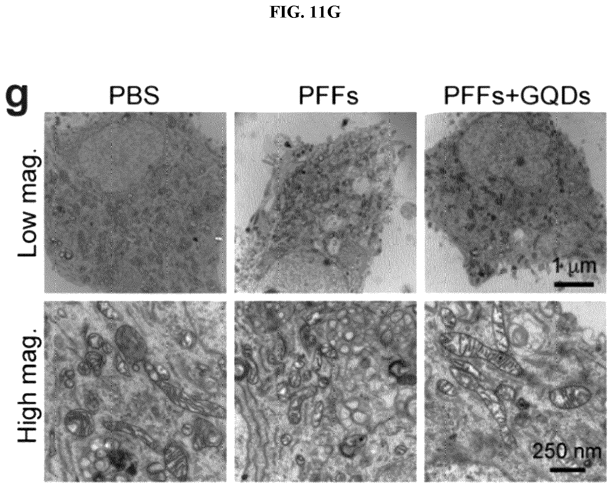

FIG. 11D illustrates representative MitoTracker-positive microscope images. 10 DIV primary cortical neurons were treated with α-syn PFFs (1 µg/ml) in the absence and presence of GQDs (1 µg/ml). After 7 days of incubation, mitochondria were labeled with MitoTracker Orange CMTMRos (red). FIG. 11E is a graph showing the results of quantifying the length of stained mitochondria (n=50 per each group), and FIG. 11F is a graph showing the aspect ratio of stained mitochondria (n=50 per each group). FIG. 11G illustrates representative TEM images of stained mitochondria at low and high magnifications. As illustrated in FIGS. 11D and 11G, it was confirmed that a reduction in the length and aspect ratio of mitochondria, induced by α-syn PFFs was recovered by GQDs.

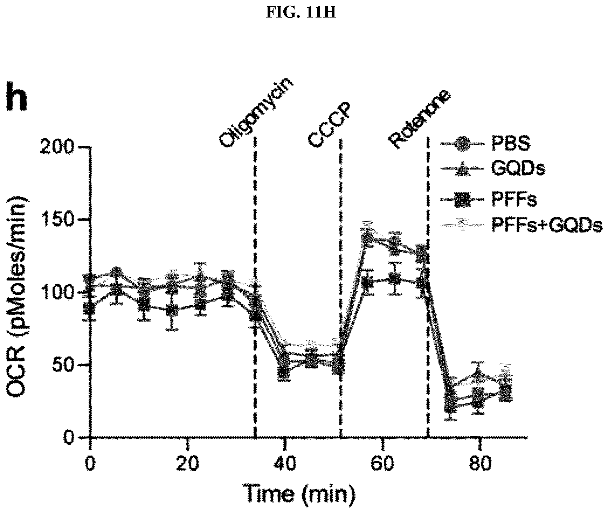

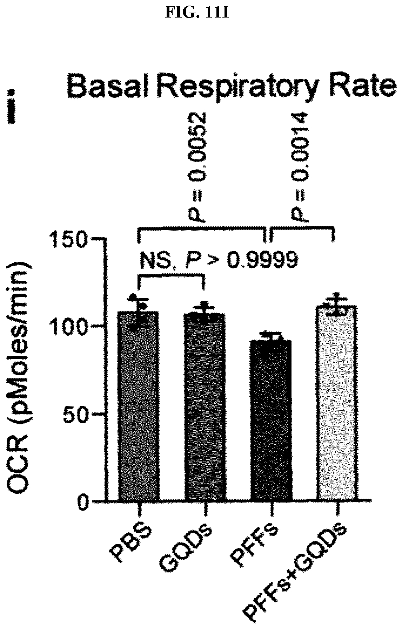

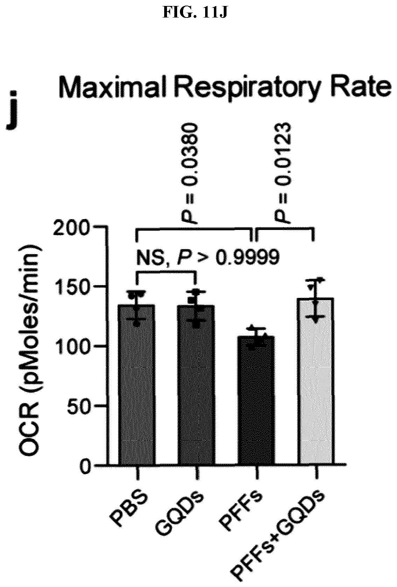

FIG. 11H is a graph showing microplate-based respirometry values for neurons. The oxygen consumption rate was measured (n=4 per each group) with an XF 24 Seahorse analyzer using primary cortical neurons treated with α-syn PFFs (1 µg/ml) in the absence and presence of GQDs (1 µg/ml) for 24 hours. FIG. 11I is a graph showing the results of quantifying the basal respiratory rate of respirometry results, and FIG. 11J is a graph showing the results of quantifying the maximal respiratory rate of respirometry results. As illustrated in FIGS. 11H to 11J, it was confirmed that an α-syn PFF-induced reduction in respiratory rate was recovered by GQDs.

Through the above results, it was confirmed that the graphene quantum dots of the present invention exhibit the effect of alleviating or treating oxidative stress, mitochondrial morphology abnormalities, and mitochondrial dysfunction, which are caused by the abnormal fibrillization and aggregation of α-syn.

### 12. Effect of GQDs on α-syn PFF-induced Primary Neuronal Toxicity and Pathology at Different Treatment Points of GQDs and Live Cell Imaging

10 DIV mouse cortical neurons were treated with α-syn PFFs (1 µg/ml) at 3 days before (n=4, Before), simultaneously with (n=4, Simul), and day 3 of incubation after treatment with GQDs (1 µg/ml) (n=4, After).

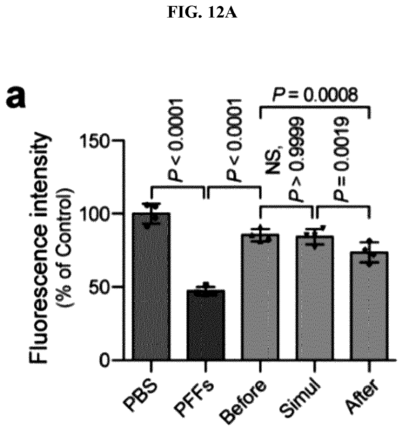

FIG. 12A illustrates alarmarBlue fluorescence analysis results. As illustrated in FIG. 12A, it was confirmed that α-syn PFF-induced cell death was inhibited by GQDs. It was confirmed that cell death was effectively inhibited in all of the case of being treated with α-syn PFFs 3 days before treatment with GQDs, the case of being treated with both α-syn PFFs and GQDs, and the case of being treated with α-syn PFFs 3 days after treatment with GQDs.

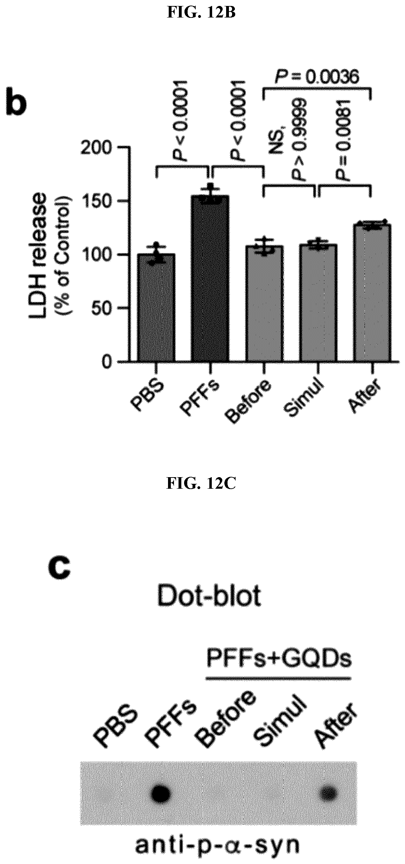

FIG. 12B is a graph showing LDH analysis results. As illustrated in FIG. 12B, it was confirmed that α-syn PFF-induced LDH secretion was inhibited by GQDs, through which it was confirmed that α-syn PFF-induced cell death was effectively inhibited by treatment with GQDs.

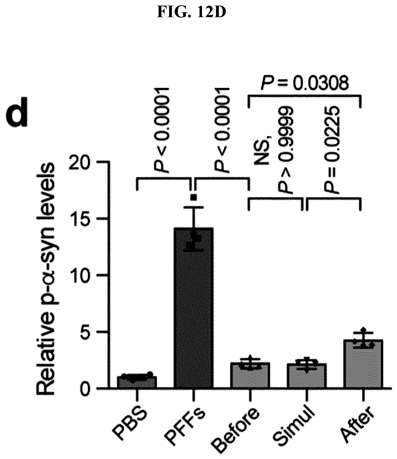

FIG.12C is a representative image of p-α-syn by dot-blot analysis, and FIG. 12D is a graph showing quantified results normalized to a PBS control (n=4 per each group). As illustrated in FIGS. 12C and 12D, it was confirmed that α-syn PFFs were effectively disaggregated by GQDs.

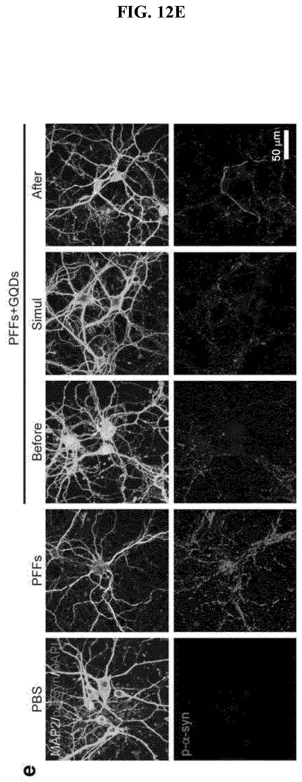

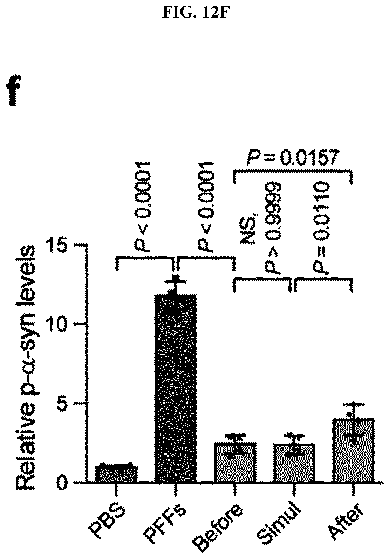

FIG. 12E illustrates representative microscope images showing p-α-syn immunostaining fluorescence by a p-α-syn antibody after 7 days of incubation, and FIG. 12F is a graph showing the results of quantifying the immunofluorescence intensity of p-α-syn normalized to a PBS control (n=4 per each group). As illustrated in FIGS. 12E and 12F, it was confirmed that α-syn PFFs were effectively disaggregated by GQDs.

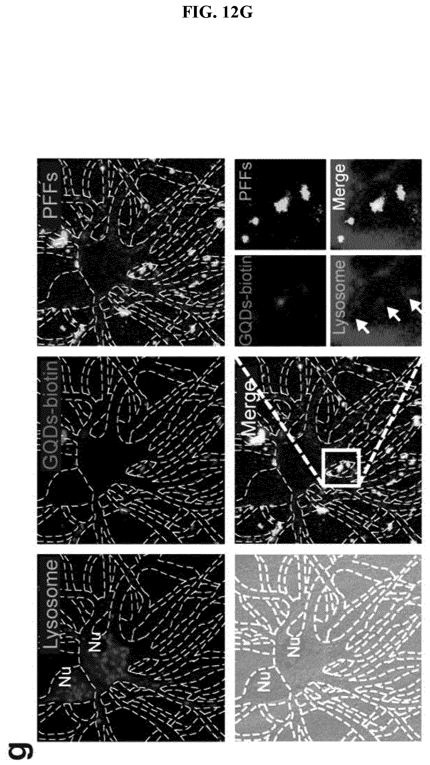

FIG. 12G illustrates live imaging results confirming that GQDs and α-syn PFFs were co-localized in the lysosomes of neurons. LysoTracker (blue), a GQDs-biotin-streptavidin Qdot complex (red), and FITC-labeled α-syn PFFs (green) were used.

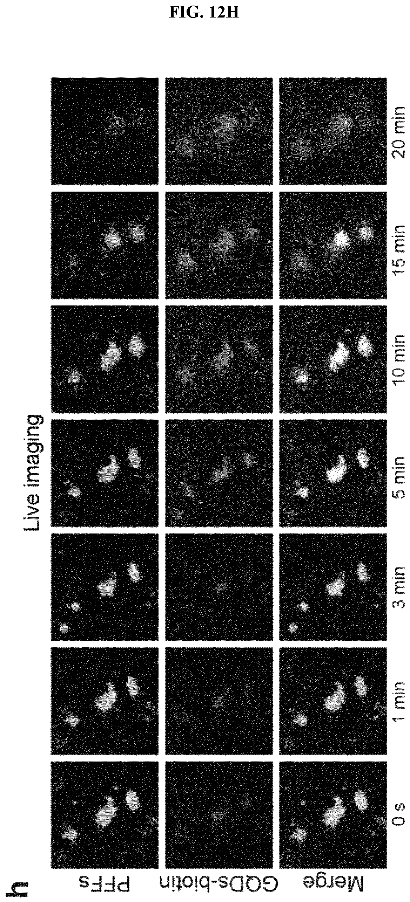

FIG. 12H illustrates a GQD-induced α-syn PFF disaggregation process showed by monitoring low-frequency fluorescence signals using live imaging.

Through the above results, it was confirmed that the graphene quantum dots of the present invention effectively disaggregated α-syn PFFs before, simultaneously with, or after the formation of α-syn PFFs. Through these results, it was confirmed that the graphene quantum dots of the present invention may be used for the prevention and treatment of diseases associated with the abnormal fibrillization or aggregation of a neuroprotein.

### 13. BBB Permeability of GQDs

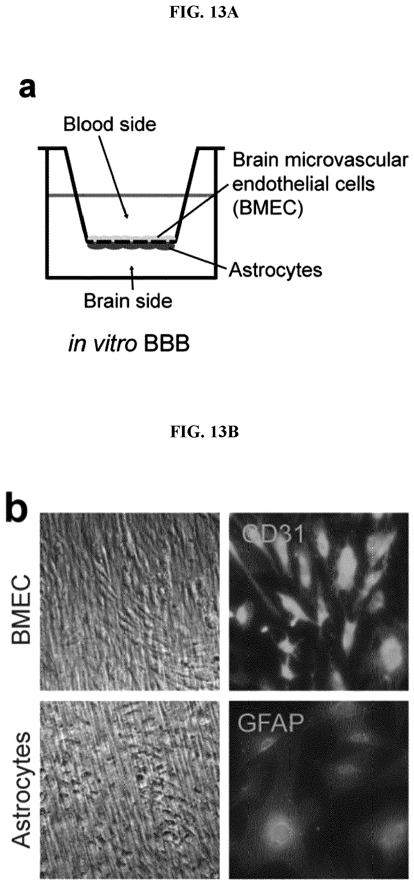

FIG. 13A illustrates an *in vitro* experiment of BBB permeability.

FIG. 13B illustrates results confirming the confluent monolayer formation of brain microvascular endothelial cells (BMECs) and astrocytes, by immunofluorescence staining with CD31 (endothelial cell marker) or GFAP (astrocyte marker) antibodies, respectively.

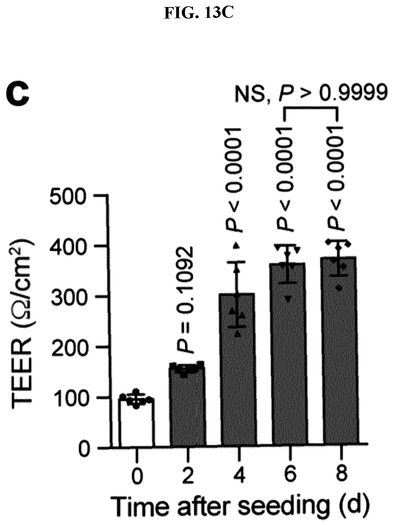

FIG. 13C illustrates the results of measuring transepithelial electrical resistance (TEER) at various time points (day 0, 2, 4, 6, and 8, n=4 per each group, *** P<0.001, one-way ANOVA with a post-hoc Bonferroni test, and error bars are standard deviation).

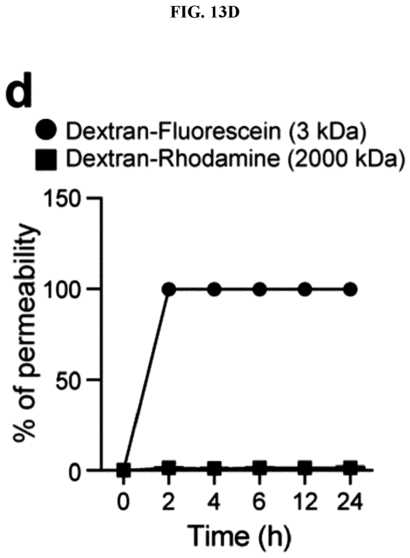

FIG. 13D illustrates the results of observing the permeability of an endothelial cell monolayer and an astrocyte by measuring the ratio between the blood side and the brain side using dextran-fluorescein (3 kDa) and dextran-rhodamine (2,000 kDa).

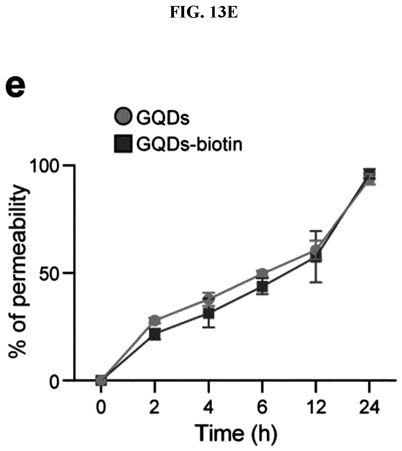

FIG. 13E is a graph showing the results of quantifying the permeability of GQDs and GQDs-biotin over time. As illustrated in FIG. 13E, it was confirmed that both GQDs and biotinylated GQDs exhibited high cell permeability.

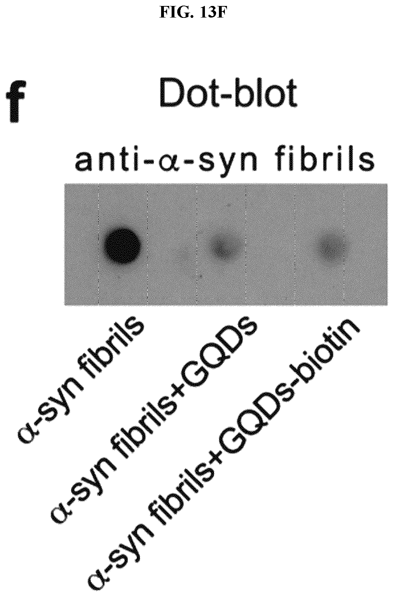

FIG. 13F is an image showing the results of observing the abilities of GQDs and GQDs-biotin to disaggregate α-syn fibrils by dot-blot. As illustrated in FIG. 13F, it was confirmed that both GQDs and biotinylated GQDs permeated into both the endothelial cell monolayer and astrocytes and effectively disaggregated α-syn fibrils.

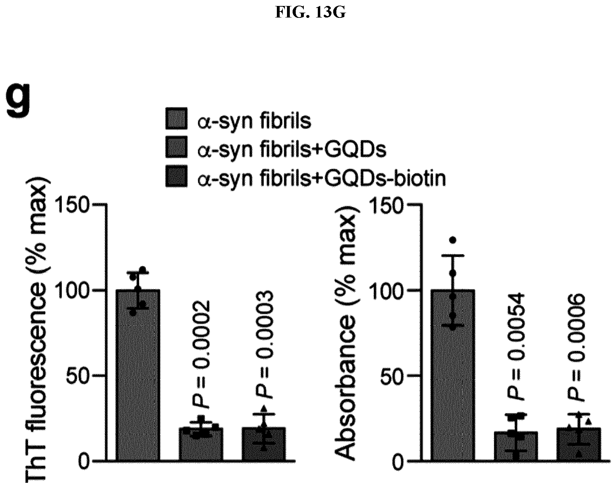

FIG. 13G is a graph showing ThT and turbidity analysis results (n=4 per each group, *** P<0.001, one-way ANOVA with a post-hoc Bonferroni test, and error bars are standard deviation). As illustrated in FIG. 13G, it was confirmed that both GQDs and biotinylated GQDs permeated into both the endothelial cell monolayer and astrocytes and effectively disaggregated α-syn fibrils, thereby effectively reducing turbidity.

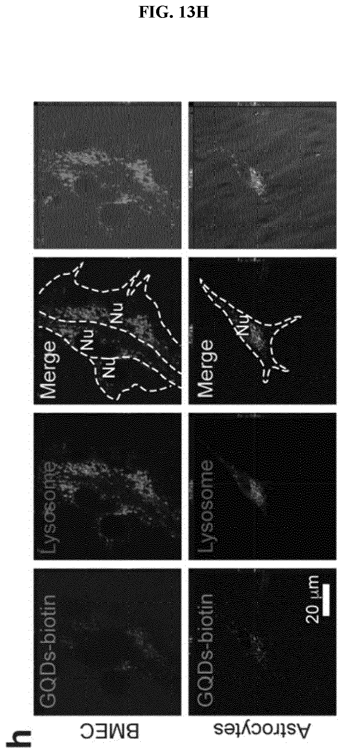

FIG. 13H illustrates confocal laser scanning microscope images of primary cultured BMECs and astrocytes after 1 hour incubation with GQDs-biotin. GQDs-biotin is shown with Qdot™streptavidin, and lysosomes are labeled by LysoTracker orange. As illustrated in FIG. 13H, it was confirmed that biotinylated GQDs were present in the lysosomes of endothelial cells and astrocytes.

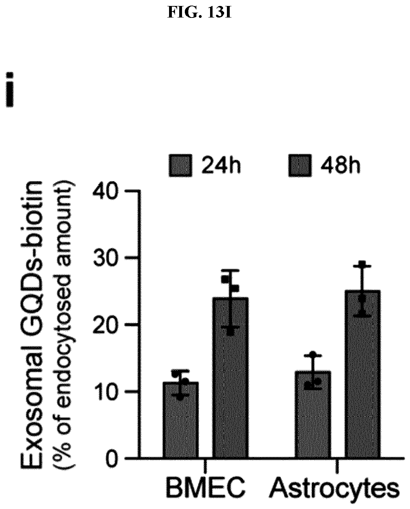

FIG. 13I is a graph showing the results of quantifying GQDs-biotin in exosomes extracted from BMECs or astrocytes. The amount of GQDs-biotin was measured using isolated exosomes at 24 and 48 hours after cell death (n=3 per each group). As illustrated in FIG. 13I, it was confirmed that, as the reaction time increased, the amount of biotinylated GQDs contained in endothelial cells and astrocytes increased.

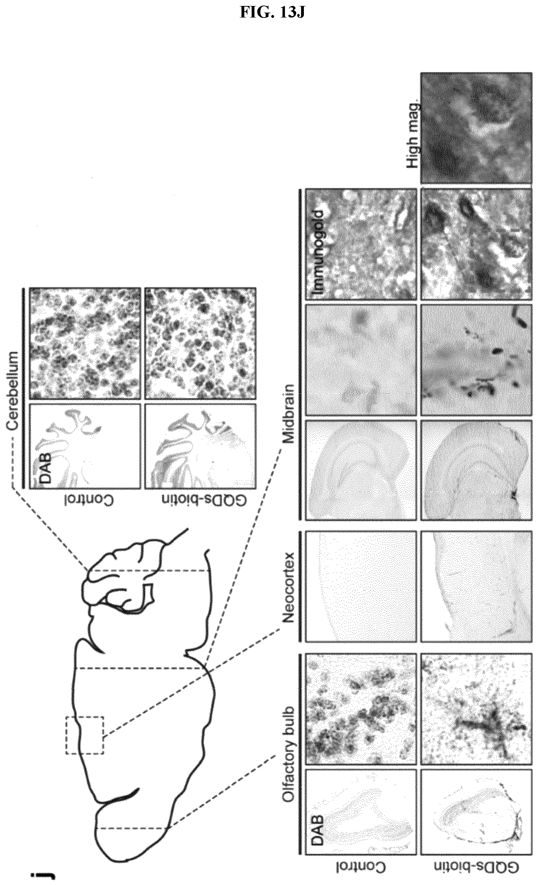

FIG. 13J illustrates images showing immunohistochemical analysis results obtained by intraperitoneally injecting GQDs-biotin (2 mg/kg) to mice, and then staining biotinylated GQDs present in the cerebellum, midbrain, olfactory bulb, and neocortex using an avidin-biotin complex detection method or an immunogold method. DAB-positive stained signals were detected at a higher level in GQDs-biotin-injected mice than that in a vehicle-injected control. Immunogold-positive signals were observed both inside neurons (red triangles) and outside neurons (blue triangles). As illustrated in FIG. 13J, it was confirmed that biotinylated GQDs also penetrated the BBB *in vivo.*

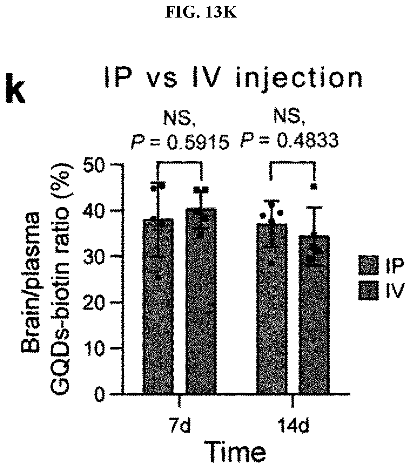

FIG. 13K is a graph showing the results of comparing intraperitoneal (i.p.; red bar) and intravenous (i.v.; blue bar) injections of GQDs-biotin. It was confirmed that both injection methods did not show any substantial difference in the results, through which it was confirmed that there was no difference in effect according to the injection method of GQDs.

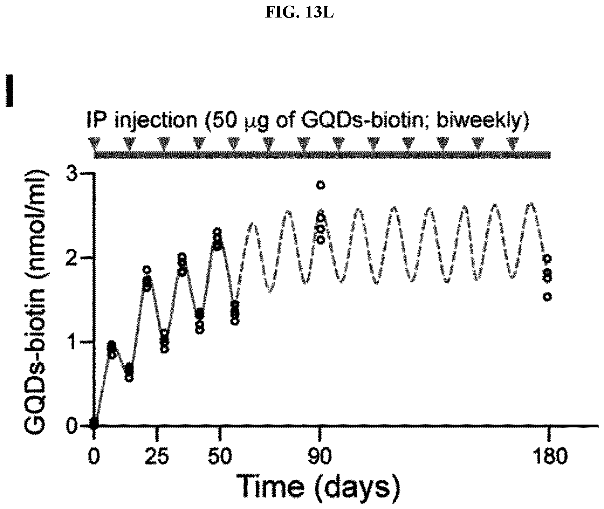

FIG. 13L illustrates the results of measuring the concentrations of GQDs-biotin using a biotin quantification kit 1 to 8 weeks, 3 months, and 6 months (n=4 per each group) after GQDs-biotin was injected biweekly. As illustrated in FIG. 13L, it was confirmed that, as the number of times of injection of biotinylated GQDs increased, the concentration of GQDs remaining in the body was also increased.

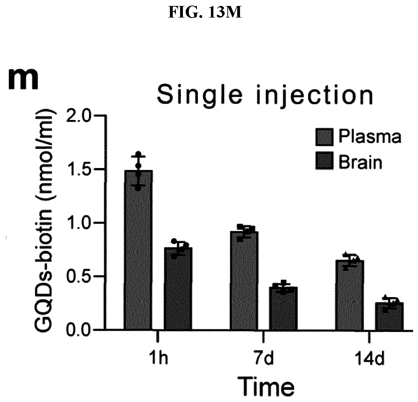

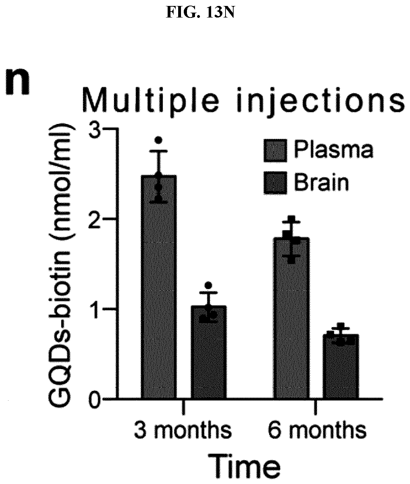

FIG. 13M is a graph showing the results of measuring concentrations in the brain (green bar) and plasma (blue bar) at 1 hour, 7 days, and 14 days (n=4 per each group) after 2 mg/kg of GQDs-biotin was intraperitoneally injected once. FIG. 13N is a graph showing the results of measuring concentrations in the brain (green bar) and plasma (blue bar) at 3 months and 6 months after multiple biweekly injections. As illustrated in FIGS. 13M and 13N, it was confirmed that GQDs remained in the body for 14 days even when injected once, and it was confirmed that GQDs remained up to 6 months when injected multiple times.

Through the above results, it was confirmed that the graphene quantum dots of the present invention not only could effectively penetrate the BBB both *in vitro* and *in vivo,* but also remained in the body for a long period of time. Through these results, it was confirmed that the graphene quantum dots of the present invention could exhibit a high therapeutic effect on diseases associated with the abnormal fibrillization or aggregation of a neuroprotein.

### 14. Effect of GQDs on α-syn PFF-induced Glial Cell Activation in Substantia Nigra (SN)

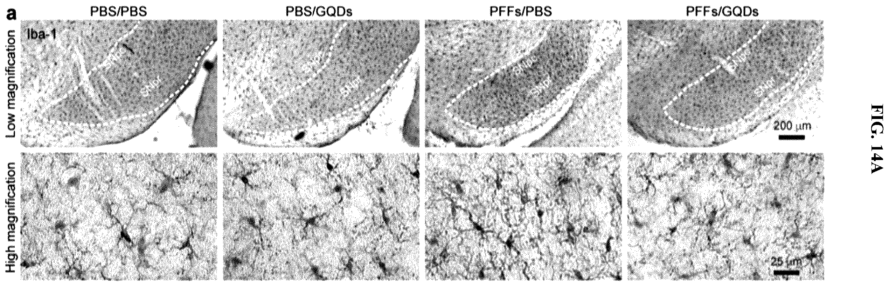

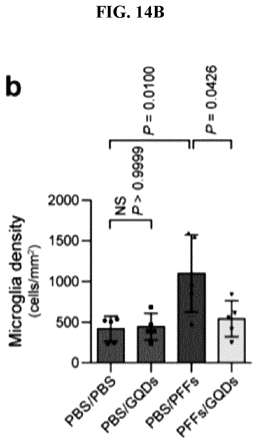

FIG. 14A illustrates representative images for Iba-1 immunohistochemical staining in the SN at low and high magnifications. Microglia, which belongs to an α-syn PFF-injected SN hemisphere, were stained with specific marker Iba-1 (ionized calcium binding protein, specific marker of microglia/macrophage). FIG. 14B is a graph showing the results of quantifying stained Iba-1-positive microglia (n=5). As illustrated in FIGS. 14A and 14B, it was confirmed that Iba-1-positive microglia increased by α-syn PFFs were inhibited by GQDs.

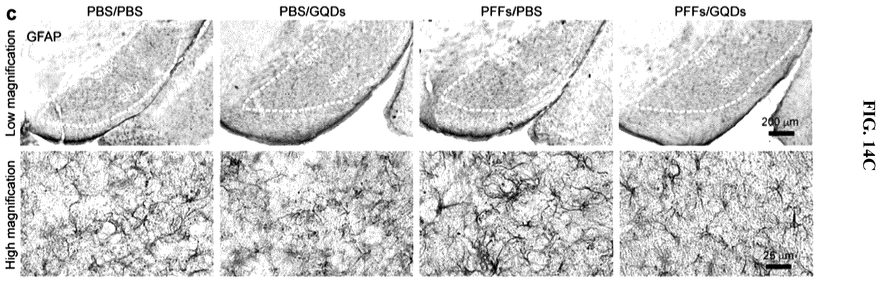

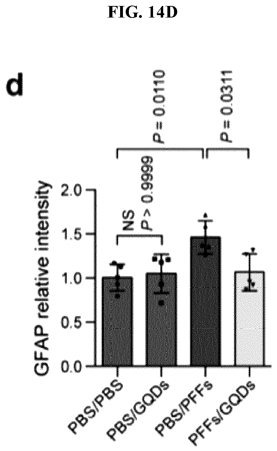

FIG. 14C illustrates representative images showing immunohistochemical staining of the GFAP in the SN at low and high magnifications. Astrocytes in the SN of an α-syn PFF-injected hemisphere were stained with anti-GFAP (glial fibrillary acidic protein; specific marker of astrocytes) antibodies. FIG. 14D is a graph showing the results of quantifying stained GFAP intensity. The GFAP intensity was normalized to a PBS-injected control (n=5 per each group) (* P <0.05; NS (not significant); 2-way ANOVA with a post-hoc Bonferroni test, and error bars are standard deviation). As illustrated in FIGS. 14C and 14D, it was confirmed that astrocytes increased by α-syn PFFs were inhibited by GQDs.

Through the above results, it was confirmed that neural inflammatory cells increased by α-syn PFFs were inhibited by GQDs.

### 15. Effect of GQDs on Glial Cell Activation in Brainstem of hA53T α-syn Transgenic Mice

To confirm the effect of GQDs of the present invention on glial cell activation in the brainstem, an experiment was conducted using the locus coeruleus (LC) of the brainstem of hA53T α-syn Tg mice.

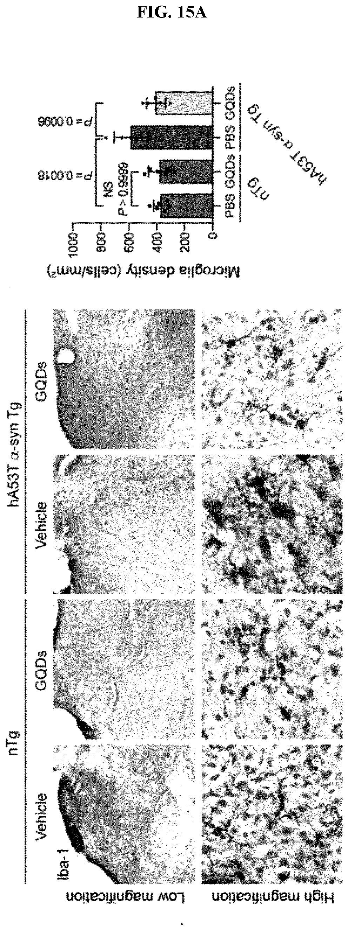

FIG. 15A illustrates immunohistochemical staining images of microglia in the LC of the brainstem at low and high magnifications and a graph showing the results of quantifying Iba-1-positive microglia (n=5 per each group, *P<0.05; **P<0.01; NS (not significant); 2-way ANOVA with a post-hoc Bonferroni test, and error bars are standard deviation). Microglia in the brainstem of nTg or hA53T α-syn Tg mice were stained with Iba-1. As illustrated in FIG. 15A, it was confirmed that the number of Iba-1-positive microglia increased in hA53T α-syn Tg mice was reduced by administration of GQDs.

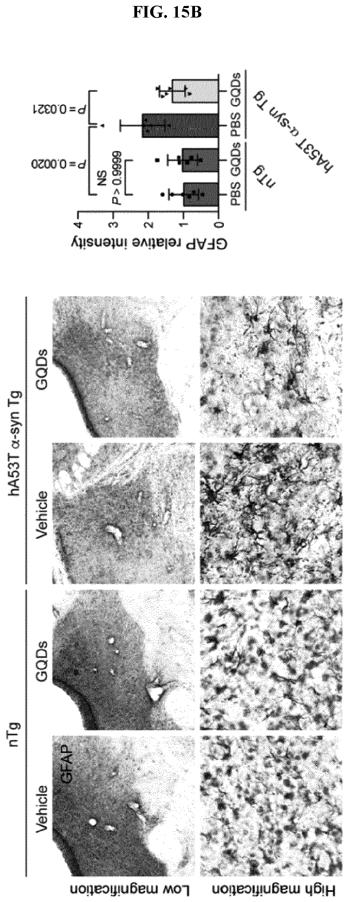

FIG. 15B illustrates immunohistochemical staining images of the GFAP in the brainstem at low and high magnifications and a graph showing the results of quantifying the stained GFAP. Relative GFAP intensities were normalized to a PBS-injected control (n=5 per each group). As illustrated in FIG. 15B, it was confirmed that the GFAP increased in hA53T α-syn Tg mice was reduced by administration of GQDs.

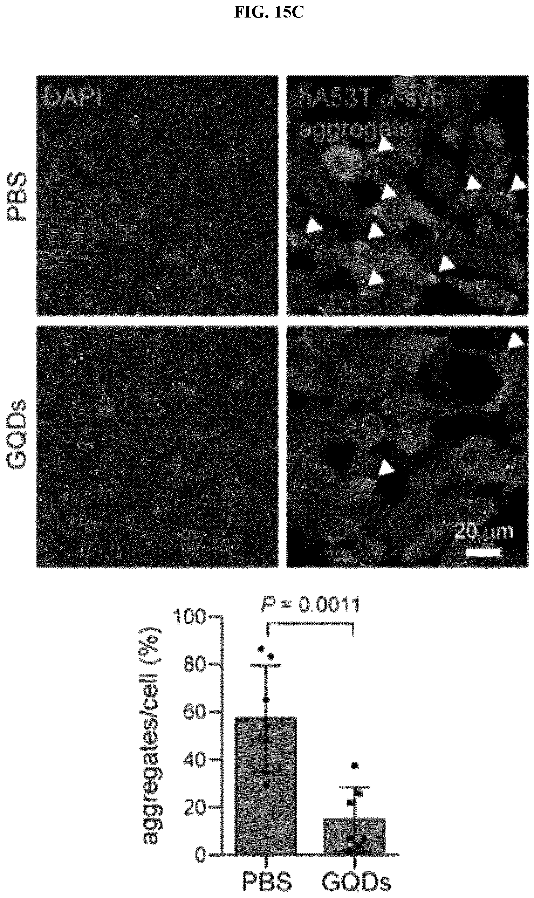

FIG. 15C illustrates images showing the results of confirming the effect of GQDs on α-syn aggregate formation in HEK293 cells with hA53T α-syn overexpression and a graph showing the results of quantifying and normalizing (n=7, **P<0.01, and error bars are standard deviation) the number of immuno-positive aggregates per field. HEK293T cells were transfected with pCMV5-myc-A53T α-syn and experimental groups were treated with GQDs (0.1 µg/ml). 48 hours after treatment with GQDs, the cells were immunostained with α-syn antibodies. White arrows indicate α-syn aggregates. As illustrated in FIG. 15C, it was confirmed that α-syn aggregate formation was effectively inhibited by GQDs.

### 16. Test of Long-term in vitro and in vivo Toxicity of GQDs

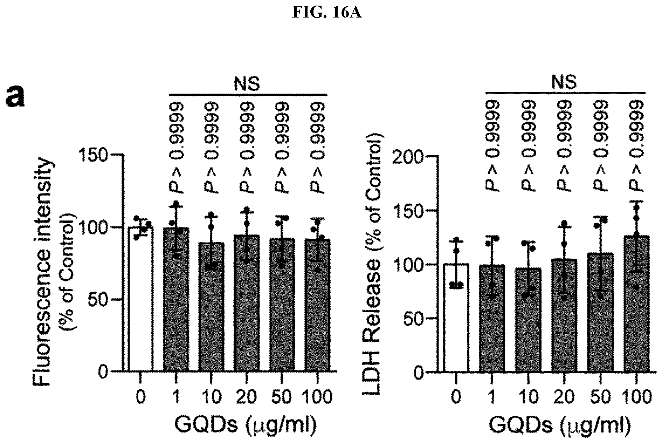

FIG. 16A illustrates graphs showing the results of measuring the cytotoxicity of GQDs in DIV 10 cortical neurons incubated for 7 days, by alamarBlue and LDH assays (n=4, at each time point, NS (not significant), one-way ANOVA with a post-hoc Bonferroni test, and error bars are standard deviation). As illustrated in FIG. 16A, it was confirmed that GQDs did not exhibit cytotoxicity when incubated for a long period of time.

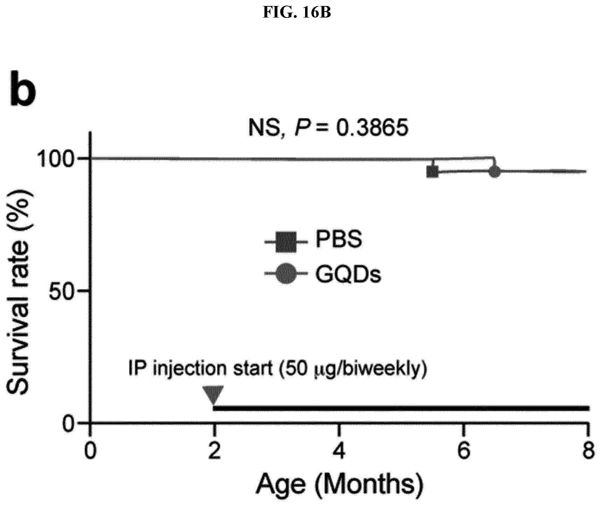

FIG. 16B is a graph showing survival curves of GQD-injected mice. 50 µg of GQDs or vehicle were intraperitoneally injected into C57BL/6 mice for 6 months and *in vivo* toxicity of GQDs as monitored (n=20 per each group). It was confirmed that there was no statistically significant difference between the GQD-injected group and the vehicle-injected control after 8 months.

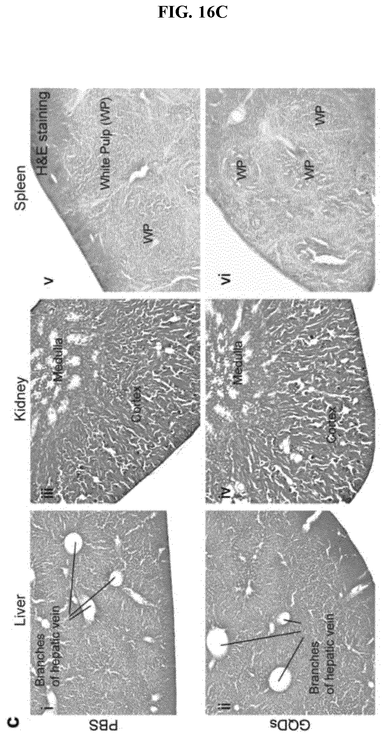

FIG. 16C illustrates images showing major organs stained with haematoxylin & eosin (H&E). As illustrated in FIG. 16C, it was confirmed that GQDs did not exhibit toxicity to major organs.

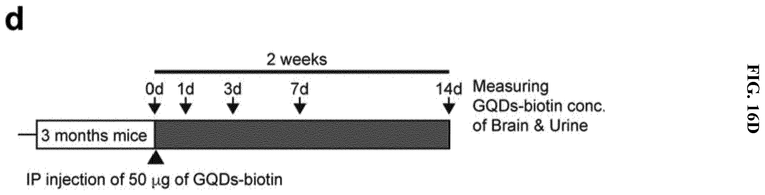

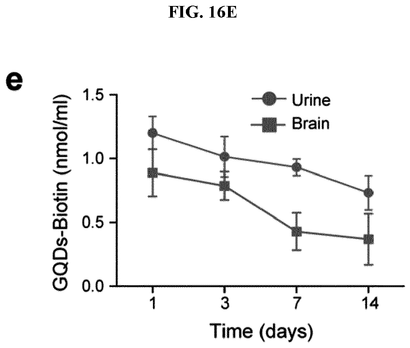

FIG. 16D is a schematic view illustrating injection and sampling methods for *in vivo* tracking of GQDs-biotin, and FIG. 16E is a graph showing the results of measuring the concentration of GQDs-biotin over time in the brain (red) or urine (blue). 50 µg of GQDs-biotin was intraperitoneally injected into C57BL/6 mice, and measurement was performed at various time points (day 1, 3, 7, and 14). As illustrated in FIG. 16E, it was confirmed that the concentration of GQDs *in vivo* was reduced since biotinylated GQDs were excreted via urine.

Through the above results, it was confirmed that the GQDs of the present invention did not exhibit *in vivo* toxicity, and after being injected, the GQDs were stably excreted via urine over time.

### 17. Comparison between nano-GOs and rGQDs on α-syn PFFs

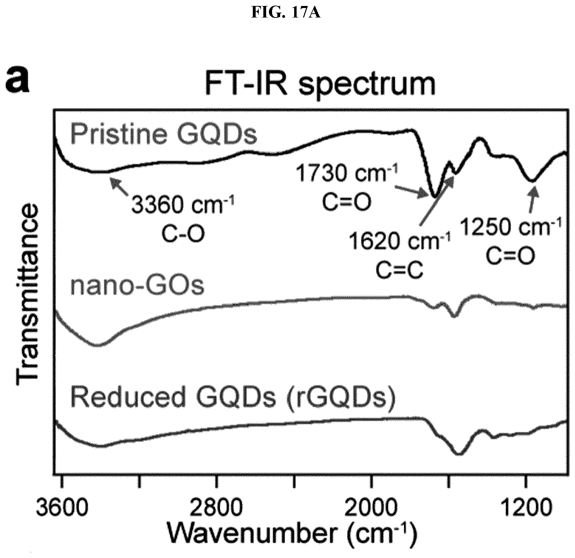

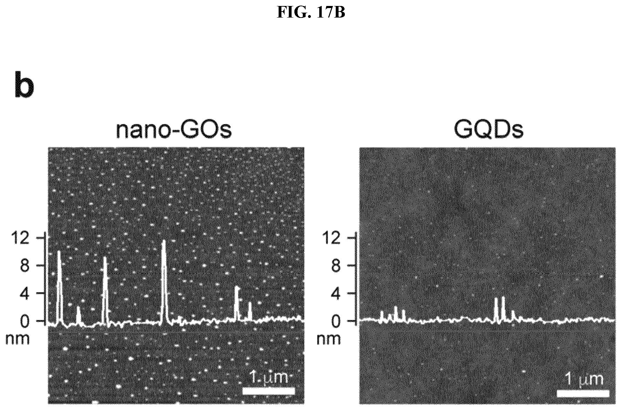

FIG. 17A is a graph showing FT-IR spectra of pristine GQDs (black), nano-GOs (nano-graphene oxides, green), and reduced GQDs (rGQDs, purple) having designated peaks for functional groups. FIG. 17B illustrates AFM images of nano-GOs and GQDs having representative line profiles. As illustrated in FIG. 17B, it was confirmed that nano-GOs exhibited an average height of about 5 nm, whereas the GQDs exhibited a height of 0.1 nm to 3 nm.

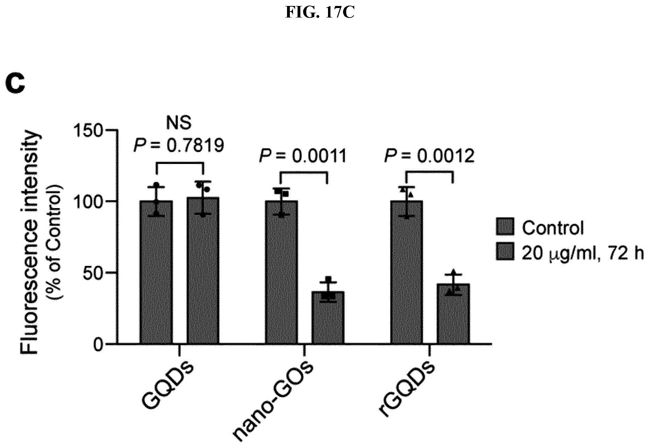

FIG. 17C is a graph showing the results of quantifying the cytotoxicity of cells after being treated with 20 µg/ml of each material and incubated for 72 hours (n=3 per each group, **P<0.01, Student's t-test, and error bars are standard deviation). As illustrated in FIG. 17C, it was confirmed that rGQDs and nano-GOs exhibited cytotoxicity, whereas GQDs did not exhibit cytotoxicity.

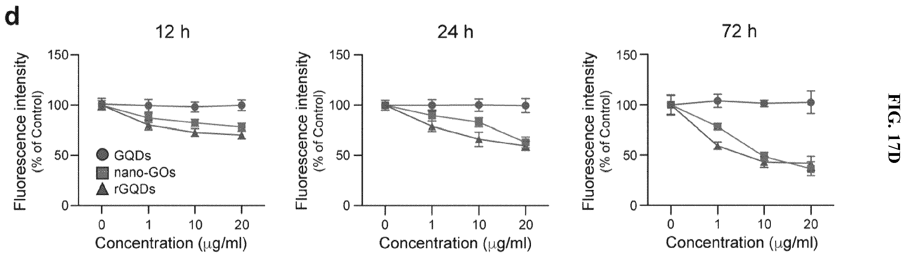

FIG. 17D illustrates graphs showing the results of monitoring cytotoxicity in SH-SY5Y cells by alarmarBlue assay 12 hours, 24 hours, and 72 hours after being treated with various concentrations (1 µg/ml, 10 µg/ml, and 20 µg/ml) of each material. As illustrated in FIG. 17D, it was confirmed that rGQDs and nano-GOs exhibited cytotoxicity, whereas GQDs did not exhibit cytotoxicity.

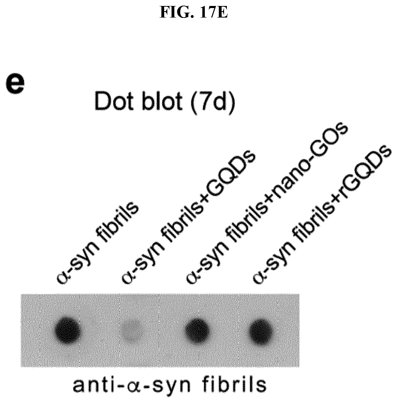

FIG. 17E is an image showing the results of comparing the α-syn PFF disaggregation effects of GQDs, nano-GOs, and rGQDs by dot blot. As illustrated in FIG. 17E, it was confirmed that rGQDs and nano-GOs had no α-syn PFF disaggregation effect.

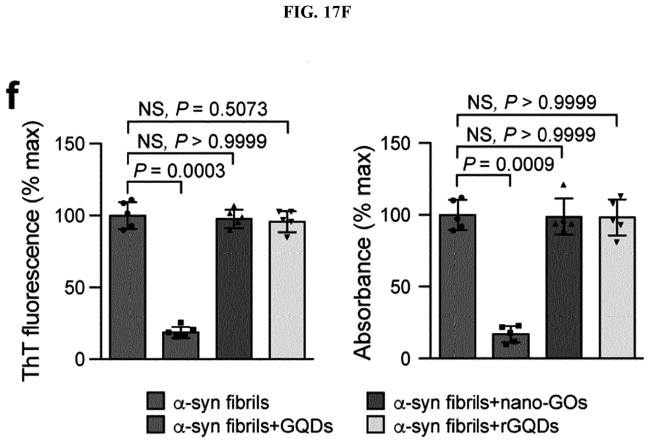

FIG. 17F illustrates graphs showing ThT and turbidity analyses (n=4 per each group, ***P <0.001; NS (not significant), one-way ANOVA with an ost-hoc Bonferroni test, and error bars are standard deviation). As illustrated in FIG. 17F, it was confirmed that GQDs effectively disaggregated α-syn PFFs, resulting in reduced turbidity and fluorescence values, whereas rGQDs and nano-GOs were unable to disaggregate α-syn PFFs.

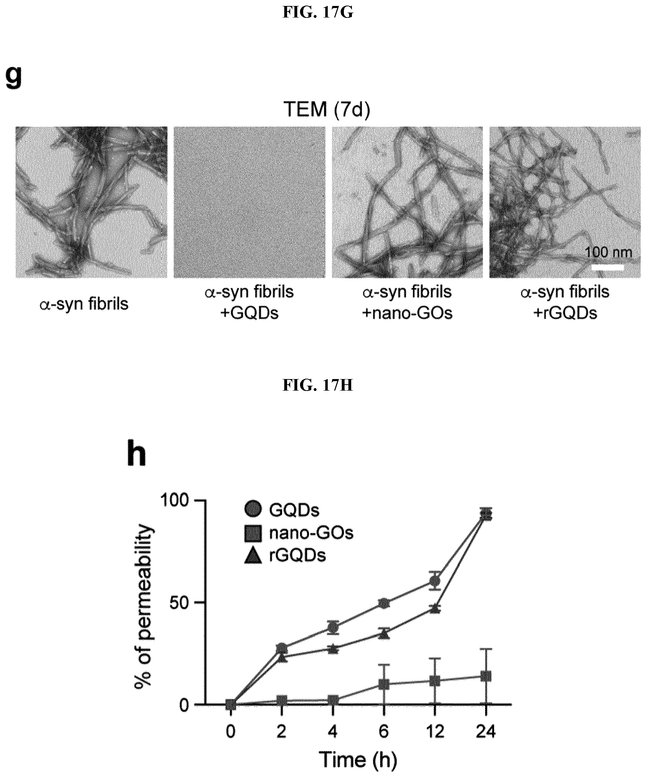

FIG. 17G illustrates TEM images acquired after 5 mg/ml of each material and α-syn PFFs were co-treated and a reaction was allowed to occur therebetween for 7 days. As illustrated in FIG. 17G, it was confirmed that rGQDs and nano-GOs were unable to disaggregate α-syn PFFs.

FIG. 17H is a graph showing the results of measuring the *in vitro* BBB permeability of GQDs, nano-GOs, and rGQDs through an *in vitro* BBB permeability experiment. As illustrated in FIG. 17H, it was confirmed that GQDs and rGQDs penetrated the BBB, whereas nano-GOs exhibited low BBB permeability.

The foregoing description of the present invention is provided for illustrative purposes, and it will be understood by those of ordinary skill in the art to which the present invention pertains that the invention may be easily modified into many different forms without departing from the technical spirit or essential characteristics of the present invention. It is therefore to be understood that the above-described embodiments are illustrative in all aspects and not restrictive.

### INDUSTRIAL APPLICABILITY

Graphene quantum dots of the present invention have no cytotoxicity, can inhibit α-syn fibrillization or disaggregate already formed α-syn fibrils, and exhibit an acting effect of penetrating the blood-brain barrier, and thus are expected to be widely used as a therapeutic agent for various diseases associated with the abnormal fibrillization or aggregation of a neuroprotein.

## Claims

1. Graphene quantum dots having negative charged surfaces, and having an average diameter of 0.5 nm to 10 nm, an average height of 0.1 nm to 3 nm, and a ratio (wt%) of carbon to oxygen of 4.0-6.5: 3.0-6.0.

2. The graphene quantum dots of claim 1, wherein the graphene quantum dots comprise carboxyl groups as terminal functional groups.

3. The graphene quantum dots of claim 2, wherein, in an FT-IR spectrum, an absorbance ratio of a -C=O peak of the carboxyl groups to an aromatic -C=C- peak is 1:1 or more.

4. The graphene quantum dots of claim 3, wherein the absorbance ratio ranges from 1:1 to 2:1.

5. The graphene quantum dots of claim 3, wherein the -C=O peak appears at 1,700 cm⁻¹ to 1,750 cm⁻¹, and the aromatic -C=C- peak appears at 1,600 cm⁻¹ to 1,650 cm⁻¹.

6. The graphene quantum dots of claim 1, wherein the average diameter ranges 1 nm to 5 nm.

7. The graphene quantum dots of claim 1, wherein the average height ranges from 0.5 nm to 2.5 nm.

8. The graphene quantum dots of claim 1, wherein the graphene quantum dots inhibit α-syn fibrillization or disaggregate α-syn fibrils.

9. The graphene quantum dots of claim 1, wherein the graphene quantum dots penetrate the blood-brain barrier (BBB).

10. The graphene quantum dots of claim 1, wherein the graphene quantum dots have no cytotoxicity.

11. A pharmaceutical composition for the prevention or treatment of a disease associated with the abnormal fibrillization or aggregation of a neuroprotein, the pharmaceutical composition comprising the graphene quantum dots according to any one of claims 1 to 10 as an active ingredient.

12. The pharmaceutical composition of claim 11, wherein the disease associated with the abnormal fibrillization or aggregation of a neuroprotein is a neurodegenerative disease, an inflammatory disease, or a metabolic disease.

13. The pharmaceutical composition of claim 12, wherein the neurodegenerative disease comprises one or more selected from the group consisting of Alzheimer's disease, Parkinson's disease, Huntington's disease, Lou Gehrig's disease, dementia, stroke, amyloidosis, fibrosis, encephalopathy, and multiple sclerosis.

14. The pharmaceutical composition of claim 12, wherein the inflammatory disease comprises one or more selected from the group consisting of erythema, atopy, rheumatoid arthritis, Hashimoto's thyroiditis, pernicious anemia, Edison's disease, type 1 diabetes, lupus, chronic fatigue syndrome, fibromyalgia, hypothyroidism, hyperthyroidism, scleroderma, Behcet's disease, inflammatory bowel disease, myasthenia gravis, Meniere's syndrome, Guilian-Barre syndrome, Sjogren's syndrome, endometriosis, psoriasis, leukoplakia, systemic scleroderma, and ulcerative colitis.

15. The pharmaceutical composition of claim 12, wherein the metabolic disease comprises one or more selected from the group consisting of diabetes, hypertension, hyperlipidemia, dyslipidemia, and non-alcoholic fatty liver.

16. A method of preventing or treating a disease associated with the abnormal fibrillization or aggregation of a neuroprotein, the method comprising administering, to an individual, a composition comprising the graphene quantum dots according to any one of claims 1 to 10 as an active ingredient.

17. A use of a composition comprising the graphene quantum dots according to any one of claims 1 to 10 as an active ingredient for the prevention or treatment of a disease associated with the abnormal fibrillization or aggregation of a neuroprotein.
